(19) Europäisches Patentamt
European Patent Office
Office européen des brevets

(11) EP 4 177 247 A1

(12) # EUROPEAN PATENT APPLICATION
published in accordance with Art. 153(4) EPC

(43) Date of publication:
**10.05.2023 Bulletin 2023/19**

(21) Application number: **21838438.6**

(22) Date of filing: **28.06.2021**

(51) International Patent Classification (IPC):
***C07D 403/14*** *(2006.01)* ***C07D 401/04*** *(2006.01)*
***C07D 487/04*** *(2006.01)* ***A61P 35/00*** *(2006.01)*
***A61K 31/4412*** *(2006.01)*

(52) Cooperative Patent Classification (CPC):
**A61K 31/4412; A61K 31/454; A61K 31/496; A61K 31/5377; A61P 17/00; A61P 25/00; A61P 29/00; A61P 35/00; A61P 35/02; A61P 37/06; C07D 401/04; C07D 401/14; C07D 403/14; C07D 487/04; C07D 491/08**

(86) International application number:
**PCT/CN2021/102873**

(87) International publication number:
**WO 2022/007659 (13.01.2022 Gazette 2022/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.07.2020 CN 202010633550**

(71) Applicant: **Beijing InnoCare Pharma Tech Co., Ltd.**
**Beijing 102206 (CN)**

(72) Inventors:
- **CHEN, Xiangyang**
  **Beijing 102206 (CN)**
- **PANG, Yucheng**
  **Beijing 102206 (CN)**

(74) Representative: **Patentanwälte Magenbauer & Kollegen**
**Partnerschaft mbB**
**Plochinger Straße 109**
**73730 Esslingen (DE)**

(54) **HETEROCYCLIC IMMUNOMODULATOR**

(57) The present invention relates to heterocyclic compounds as immunomodulators, or pharmaceutically acceptable salts thereof. Specifically, the present invention relates to compounds as represented by general formula (I) and pharmaceutically acceptable salts thereof. The present invention also relates to methods for preparing the compounds or the pharmaceutically acceptable salts thereof. The compounds can be used for treating and/or preventing tumors, inflammations or immune diseases.

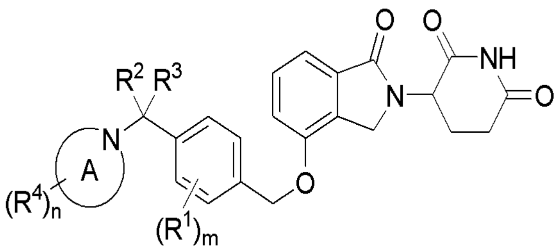

(I)

EP 4 177 247 A1

## Description

## Technical Field

The present invention relates to a heterocyclic compound as an immunomodulator, or a pharmaceutically acceptable salt thereof. The present invention also relates to a method for preparing said compound or a pharmaceutically acceptable salt thereof. The present invention further relates to the use and the using method of the compound or the pharmaceutically acceptable salt thereof in the aspects of immunoregulation, cancer resistance, anti-inflammation and the like.

## Background technology

The "lidomide"-compounds such as lenalidomide are immunomodulators (immunomodulatory drug; IMID) and have multiple action mechanisms. On the molecular level, this kind of immunomodulators regulates the function of a $CRL4^{CRBN}$-E3 ubiquitin ligase complex by combining the E3 ubiquitin ligase, causes Ikaros (IKZF1), Aiolos (IKZF3), protein kinase CK1$\alpha$ (CK1$\alpha$), transformation termination factor GSPT1 and the like to be ubiquitinated and degraded by 26S proteasome, thereby changing the secretion of various cytokines (such as IL-2, IL-6, IL-10, TNF$\alpha$, and IL-1$\beta$) and influencing the activity of immune cells. After different "lidomide"-compounds are combined with the $CRL4^{CRBN}$-E3 ubiquitin ligase complex, different substrate protein degradation specificities are shown, so that different indications are provided. Lenalidomide and pomalidomide both can induce cereblon (CRBN) to degrade the transcription factors Ikaros and Aiolos, which play important roles in blood development and differentiation, and are used to treat multiple myeloma; however, only lenalidomide can cause CK1$\alpha$ to degrade and is used for treating myelodysplastic syndrome associated with 5q deficiency. In addition, CC-885 promotes the degradation of GSPT1, but neither lenalidomide nor pomalidomide has such an action. In the non-immunoregulation, the "lidomide"-compounds could inhibit the angiogenesis of tumor cells by inhibiting the vascular endothelial growth factor (VEGF), and could also directly inhibit the proliferation of tumor cells and induce the decomposition of abnormal cells.

Through the regulation of immunity and non-immunity, the"lidomide"-compounds have received extensive attention in the treatment of many types of malignant tumors and immune diseases, such as multiple myeloma, myelodysplastic syndrome, hematological tumors/solid tumors (for example lymphoma, non-small cell lung cancer, pancreas, prostate, brain, kidney, ovary, and the like), and systemic lupus erythematosus. In addition, the "lidomide"-compounds can be combined with other drugs for treating diseases, such as small molecule targeted drugs and chemotherapeutic drugs, and macromolecule medicines (such as PD-1 antibody, CD20 antibody, CD19 antibody and the like). With the further development of the research and development of the compounds in the aspects of immunoregulation, cancer resistance, anti-inflammation and the like, the clinical indications of the compounds are also continuously increased. Although the chemical structures of the "lidomide"-compounds are similar, they have different action mechanisms, clinical treatment effects, and toxicity and side effects. The clinical common side effects of thalidomide, such as neurological diseases, constipation and sleepiness, are rare in lenalidomide; pomalidomide does not have the side effect of rash in lenalidomide. Therefore, there is a need to develop new immunomodulators and expand indications to meet different clinical needs. The present invention relates to a compound having a structure shown in general formula (I), which is used as an immunomodulator and is combined with the E3 ubiquitin ligase to effectively regulate the expression and/or biological functions of proteins such as IL-2, IL-6, IL-10, TNF$\alpha$, VEGF and the like.

## Summary of the Invention

## Definition

Unless otherwise stated to the contrary, the following terms used in the specification and claims have the following meanings.

"$C_{x-y}$" represents the range of carbon atoms, wherein x and y are integers, for example, $C_{3-8}$cycloalkyl represents a cycloalkyl with 3-8 carbon atoms, that is, a cycloalkyl with 3, 4, 5, 6, 7 or 8 carbon atoms. It should also be understood that "$C_{3-8}$" also includes any subranges therein, for example $C_{3-7}$, $C_{3-6}$, $C_{4-7}$, $C_{4-6}$, $C_{5-6}$, and the like.

"Alkyl" refers to a saturated straight or branched chain hydrocarbyl group containing 1 to 20 carbon atoms, for example 1 to 8 carbon atoms, 1 to 6 carbon atoms or 1 to 4 carbon atoms. Non-limiting examples of alkyl include methyl, ethyl, n-propyl, iso-propyl, n-butyl, iso-butyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, and the like.

"Alkylene" refers to a saturated straight or branched chain hydrocarbon divalent group containing 1 to 20 carbon atoms, for example 1 to 6 carbon atoms or 1 to 4 carbon atoms. Non-limiting examples of alkylene include $-CH_2-$,

$-CH(CH_3)-$, $-CH_2CH_2-$, $-CH_2CH_2CH_2-$, $-(CH_3)C(CH_3)-$, $-CH_2CH_2CH_2CH_2-$, $-CH_2CH(CH_3)CH_2-$ and the like.

"Cycloalkyl" refers to a saturated cyclic hydrocarbyl substituent containing 3 to 14 carbon ring atoms. Cycloalkyl can be a monocyclic carbon ring, typically containing 3 to 8, 3 to 7, or 3 to 6 carbon ring atoms. Non-limiting examples of monocyclic cycloalkyl include cyclopropyl, cyclobutyl, cyclopentyl, cyclohexyl, cycloheptyl, and the like. Cycloalkyl can also be a bi- or tricyclic ring that is fused, bridged or spiro, such as decahydronaphthalenyl, bicyclo[2.2.2]octane, spiro[3.3]heptane, and the like.

"Heterocyclyl or heterocycle" refers to a saturated or partially unsaturated monocyclic or polycyclic cyclic group comprising 3 to 20 ring atoms, for example 3 to 14, 3 to 12, 3 to 10, 3 to 8, 3 to 6, or 5 to 6 ring atoms, one or more of which are selected from nitrogen, oxygen, or $S(O)_m$ (where m is an integer of 0 to 2), but excluding ring moieties of -O-O-, -O-S-, or -S-S-, the remaining ring atoms being carbon. Preferably it comprises 3 to 12 ring atoms, more preferably 3 to 10 ring atoms, more preferably 4 to 7 ring atoms, more preferably 4 to 6 ring atoms, most preferably 5 or 6 ring atoms, of which 1 to 4 are heteroatoms, more preferably of which 1 to 3 are heteroatoms, and most preferably of which 1 to 2 are heteroatoms. Non-limiting examples of monocyclic heterocyclyl include pyrrolidinyl, oxetanyl, piperidinyl, piperazinyl, tetrahydrofuranyl, tetrahydropyranyl, tetrahydrothiopyranyl, morpholinyl, thiomorpholinyl, homopiperazinyl, azetidinyl, and the like. Non-limiting examples of polycyclic heterocyclyl include fused, bridged or spiro polycyclic heterocyclic groups, such as octahydrocyclopenta[c]pyrrole, octahydropyrrolo[1,2-a]pyrazine, 3,8-diazabicyclo[3.2.1]octane, 5-azaspiro[2.4]heptane, 2-oxa-7-azaspiro[3.5]nonane, and the like.

"Aryl or aromatic ring" refers to an aromatic monocyclic or a fused polycyclic group containing 6 to 14 carbon atoms, preferably being 6- to 10-membered, for example phenyl and naphthyl, most preferably phenyl. The aryl ring may be fused to a heteroaryl, heterocyclyl or cycloalkyl ring, where the ring attached to the parent structure is the aryl ring, and its non-limiting examples include:

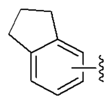 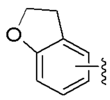 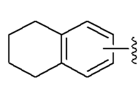 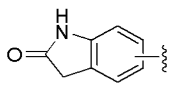 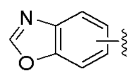 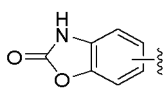 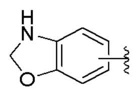

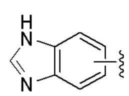 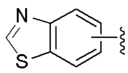 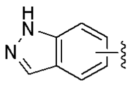 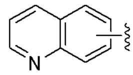 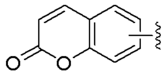 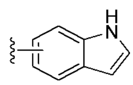

and the like.

"Heteroaryl or heteroaromatic ring" refers to a heteroaromatic system comprising 5 to 14 ring atoms, of which 1 to 4 ring atoms are selected from heteroatoms including oxygen, sulfur and nitrogen. Heteroaryl is preferably 5- to 10-membered, more preferably heteroaryl is 5- or 6-membered, for example furyl, thienyl, pyridinyl, pyrrolyl, pyrimidinyl, pyrazinyl, pyrazolyl, imidazolyl, tetrazolyl, oxazolyl, isoxazolyl, thiazolyl, isothiazolyl, quinolinyl, isoquinolyl, indolyl, isoindolyl, and the like. The heteroaryl ring may be fused to an aryl, heterocyclyl or cycloalkyl ring, where the ring attached to the parent structure is the heteroaryl ring, and its non-limiting examples include:

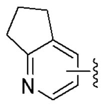 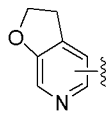 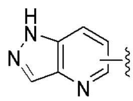 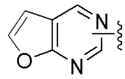 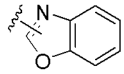 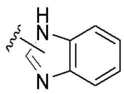

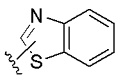 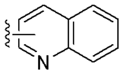 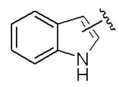

and the like.

"Halogen" refers to fluorine, chlorine, bromine or iodine.

"Cyano" refers to -CN.

"Oxo" refers to =O.

"Carbonyl" refers to a -C(O)- group.

"Sulfonyl" refers to a $-S(O)_2-$ group.

"Sulfinyl" refers to a -S(O)- group.

"Optionally" means that the subsequently described event or circumstance can but need not occur, and that the expression includes instances where the event or circumstance occurs or does not occur. For example, a "heterocyclic

group optionally substituted with an alkyl group" means that an alkyl group may but need not be present, and the expression includes cases where the heterocyclic group is substituted with an alkyl group and cases where the heterocyclic group is not substituted with an alkyl group. "Substitution"refers to one or more hydrogen atoms, preferably 5, more preferably 1 to 3 hydrogen atoms in a group are independently substituted with a corresponding number of substituents. It goes without saying that substituents are only in their possible chemical positions and that a person skilled in the art can determine (by experiment or theory) possible or impossible substitutions without undue effort. For example, an amino or hydroxyl group with free hydrogen may be unstable when bonded to a carbon atom with an unsaturated (e.g., ethylenic) bond. The substituents include, but are not limited to, halogen, cyano, nitro, oxo, $-SF_5$, $C_{1-4}$alkyl, $C_{3-7}$cycloalkyl, 4- to 7-membered heterocyclyl, phenyl, 5- to 6-membered heteroaryl and the like.

"Isomer" refers to compounds that have the same molecular formula but differ in the nature or sequence of bonding of their atoms or in the spatial arrangement of their atoms. Isomers that differ in the spatial arrangement of their atoms are referred to as "stereoisomers". Stereoisomers include optical isomers, geometric isomers and conformational isomers.

The compounds of the present invention may exist in the form of optical isomers. Optical isomers include enantiomers and diastereoisomers. Enantiomers are two stereoisomers that are mirror images of each other but are not superimposable. A racemic mixture or racemate refers to a mixture of equal amounts of the left- and right-handed enantiomers of a chiral molecule. Diastereoisomers mean that two stereoisomers are not mirror images of each other and are not superimposable. When the optical isomer is a single isomer and its absolute configuration is determined, according to the configuration of the substituent on the chiral carbon atom, it is the absolute configuration of "R"or "S"; when the absolute configuration of the optical isomer is not determined, it is (+) or (-) according to the measured optical rotation. Methods of preparing and separating optical isomers are known in the art.

The compounds of the present invention may also exist as geometric isomers. The present invention contemplates various geometric isomers and mixtures thereof resulting from the distribution of substituents around the carbon-carbon double bond, the carbon-nitrogen double bond, the cycloalkyl or the heterocyclic group. Substituents around the carbon-carbon double bond or the carbon-nitrogen bond are assigned Z or E configurations, and substituents around the cycloalkyl or the heterocycle are assigned cis or trans configurations.

The compounds of the present invention may also exhibit tautomerism, e.g. keto-enol tautomerism.

It should be understood that the present invention includes any tautomeric or stereoisomeric form and mixtures thereof, and is not limited to any one tautomeric or stereoisomeric form used in the nomenclature or chemical structural formula of the compound.

"Isotopes" refer to all isotopes of atoms occurring in the compounds of the present invention. Isotopes include those atoms having the same atomic number but different mass numbers. Examples of isotopes suitable for incorporation into compounds of the present invention are hydrogen, carbon, nitrogen, oxygen, phosphorus, sulfur, fluorine and chlorine, for example, but not limited to, $^{2}H$ (D), $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$. Isotopically-labeled compounds of the present invention can generally be prepared by conventional techniques known to those skilled in the art or by methods analogous to those described in the appended Examples using appropriate isotopically-labeled reagents in place of non-isotopically-labeled reagents. Such compounds have various potential uses, for example as standards and reagents in assays of biological activity. In the case of stable isotopes, such compounds have the potential to advantageously alter biological, pharmacological or pharmacokinetic properties. Deuterium (D) is the preferred isotope of the present invention, e.g. hydrogen in methyl, methylene or methine may be replaced by deuterium.

The compounds of the present invention may be administered in the form of prodrugs. "Prodrug" refers to a derivative that is converted into a biologically active compound of the present invention under physiological conditions in vivo, such as by oxidation, reduction, hydrolysis, or the like (each of which is carried out using an enzyme or without the participation of an enzyme). Examples of prodrugs include the following compounds in which the amino group in the compound of the present invention is acylated, alkylated or phosphorylated, for example eicosanoylamino, alanylamino, pivaloyloxymethylamino, or in which the hydroxyl group is acylated, alkylated, phosphorylated or converted to borate, e.g. acetoxy, palmitoyloxy, pivaloyloxy, succinyloxy, fumaryloxy, alanyloxy, or in which the carboxyl group is esterified or amidated, or in which the sulfhydryl group forms a disulfide bridge with a carrier molecule, such as a peptide, that selectively delivers the drug to the target and/or to the cytosol of the cell. These compounds can be prepared from the compounds of the present invention according to known methods. "Pharmaceutically acceptable salt" refers to salts prepared from pharmaceutically acceptable bases or acids, including inorganic bases or acids and organic bases or acids. In case the compounds of the present invention contain one or more acidic or basic groups, the present invention also includes their corresponding pharmaceutically acceptable salts. Compounds according to the present invention which contain acidic groups can thus exist in salt form and can be used according to the present invention, for example as alkali metal salts, alkaline earth metal salts or ammonium salts. More specific examples of such salts include sodium salt, potassium salt, calcium salt, magnesium salt or salts with ammonia or organic amines such as ethylamine, ethanolamine, triethanolamine or amino acids. The compounds according to the present invention which contain basic groups can exist in the form of salts and can be used according to the present invention in the form of their addition salts

with inorganic or organic acids. Examples of suitable acids include hydrochloric acid, hydrobromic acid, phosphoric acid, sulfuric acid, nitric acid, methanesulfonic acid, p-toluenesulfonic acid, naphthalene disulfonic acid, oxalic acid, acetic acid, tartaric acid, lactic acid, salicylic acid, benzoic acid, formic acid, propionic acid, pivalic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid and other acids known to those skilled in the art. If the compounds according to the present invention simultaneously contain acidic and basic groups in the molecule, the present invention also includes, in addition to the salt forms mentioned, inner salts or betaines. The individual salts can be obtained by conventional methods known to those skilled in the art, for example by contacting these with organic or inorganic acids or bases in a solvent or dispersant or by anion exchange or cation exchange with other salts.

"Pharmaceutical composition" refers to compositions containing one or more compounds of the present invention or pharmaceutically acceptable salts, stable isotope derivatives, isomers, prodrugs or mixtures thereof and other components such as pharmaceutically acceptable carriers and excipients. The purpose of the pharmaceutical composition is to promote the administration to the organism, facilitate the absorption of the active ingredient and thus exert biological activity.

Therefore, when referring to "a compound", "a compound of the present invention" or "the compound of the present invention" in this application, all forms of said compounds are included, e.g. pharmaceutically acceptable salts, stable isotope derivatives, isomers, prodrugs or mixtures thereof.

"Cancer/tumor" includes but is not limited to digestive tract/ gastrointestinal tract cancer, colon cancer, liver cancer, skin cancer (including mastocytoma and squamous cell carcinoma), breast cancer, ovarian cancer, prostate cancer, lymphoma, leukemia (including acute myeloid leukemia and chronic myelogenous leukemia), kidney cancer, lung cancer, muscle cancer, bone cancer, bladder cancer, brain cancer, melanoma (including oral and metastatic melanoma), Kaposi's sarcoma (including myeloma of multiple myeloma), myeloproliferative disease, proliferative diabetic retinopathy, vascular proliferation-related diseases/tumors, and the like.

"Inflammatory disease" includes, but is not limited to, arthritis, Hashimoto's thyroiditis, autoimmune hemolytic anemia, autoimmune atrophic gastritis with pernicious anemia, autoimmune encephalomyelitis, autoimmune orchitis, Goodpasture's disease, autoimmune thrombocytopenia, sympathetic ophthalmia, myasthenia gravis, Graves' disease, primary biliary cirrhosis, hepatitis, primary sclerotic cholangitis, chronic aggressive hepatitis, non-alcoholic fatty liver disease, non-alcoholic fatty hepatitis, ulcerative colitis, membranous glomerulopathy, systemic lupus erythematosus, rheumatoid arthritis, psoriatic arthritis, Sjogren syndrome, Reiter syndrome, polymyositis, dermatomyositis, Type I interferon diseases, including Aicardi-Goutières syndrome and other systemic sclerosis that overexpress type I interferon, Mendelian diseases, polyarteritis nodosa, multiple sclerosis, relapsing-remitting multiple sclerosis, primary progressive multiple sclerosis, secondary progressive multiple sclerosis, bullous pemphigoid; additional O-cell (humoral) or T-cell based autoimmune diseases, including Cogan's syndrome, ankylosing spondylitis, Wegener's granulomatosis, autoimmune alopecia, type I or juvenile diabetes, thyroiditis.

A "therapeutically effective amount" refers to include an amount of the compound of the present invention that is effective in treating or preventing the disease.

"Patient" refers to a mammal, especially a human.

The present invention relates to a compound represented by general formula (I), or a pharmaceutically acceptable salt, a stable isotope derivative, an isomer thereof, or a prodrug thereof

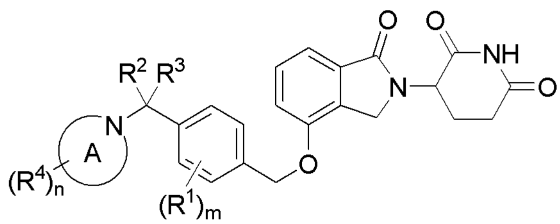

(I)

wherein:

ring A is a nitrogen-containing 4- to 10-membered heterocycle;

$R^1$ is D or halogen;

$R^2$ and $R^3$ are each independently selected from H, D, halogen, cyano, $C_{1-6}$alkyl, $C_{3-6}$cycloalkyl or 3- to 8-membered heterocyclyl, but both cannot be H and/or D at the same time, or $R^2$ and $R^3$ together with the carbon atoms attached thereto form $C_{3-6}$cycloalkane or 3- to 8-membered heterocycle;

$R^4$ is D, halogen, cyano, oxo, $C_{1-6}$alkyl, $C_{3-6}$cycloalkyl, $-OC_{1-6}$alkyl, $-C(O)C_{1-6}$alkyl, $-C(O)C_{3-6}$cycloalkyl, $-S(O)_2C_{1-6}$alkyl, $-S(O)_2C_{3-6}$cycloalkyl, aryl or heteroaryl, wherein one or more of the alkyl, the cycloalkyl, the aryl

and the heteroaryl is/are optionally substituted with D, halogen, cyano, $C_{1-2}$alkyl or fluoro$C_{1-2}$alkyl;
m is an integer of 0-4; and
n is an integer of 0-2.

In an embodiment, ring A is a 4- to 10-membered monocyclic heterocycle containing one N atom, or a fused-, bridged- or spiro-bicyclic heterocycle (such as morpholine, piperidine, thiomorpholine-1,1-dioxide, 2-oxa-5-azabicyclo[2.2.1]heptane, or the like); $R^4$ is D, halogen, oxo, $-CF_3$ or $C_{1-6}$alkyl.

In an embodiment, ring A is a 6- to 10-membered monocyclic heterocycle containing two N atoms, or a fused-, bridged- or spiro-bicyclic heterocycle (such as piperazine, 3,6-diazabicyclo[3.1.1]heptane, 2,6-diazaspiro[3.3]heptane, or the like); $R^4$ is attached to the second N atom and is $C_{1-6}$alkyl, $C_{3-6}$cycloalkyl, $-C(O)C_{1-6}$alkyl, $-C(O)C_{3-6}$cycloalkyl, $-S(O)_2C_{1-6}$alkyl, $-S(O)_2C_{3-6}$cycloalkyl, phenyl or 5- to 6-membered heteroaryl containing N, O and/or S, wherein one or more hydrogens of the alkyl, the cycloalkyl, the phenyl and the heteroaryl are optionally substituted with D, halogen, cyano, $C_{1-2}$alkyl or fluoro$C_{1-2}$alkyl.

In a preferable embodiment, ring A is piperazine; $R^4$ is attached to the second N atom and is $C_{1-6}$alkyl, $C_{3-6}$cycloalkyl, $-C(O)C_{1-6}$alkyl, $-C(O)C_{3-6}$cycloalkyl, $-S(O)_2C_{1-6}$alkyl, phenyl, pyridinyl or pyrimidinyl, wherein one or more hydrogens of the alkyl, the cycloalkyl, the phenyl, the pyridinyl or the pyrimidinyl are optionally substituted with D, halogen, cyano or $C_{1-2}$alkyl; n is 0 or 1.

In an embodiment, $R^2$ is H; $R^3$ is cyano, $C_{1-6}$alkyl or $C_{3-6}$cycloalkyl.

In an embodiment, both $R^2$ and $R^3$ are methyl.

In an embodiment, $R^2$ and $R^3$ together with the carbon atoms attached thereto form $C_{3-6}$cycloalkane.

In a preferable embodiment, m is 0.

In some embodiments, the compound represented by general formula (I) is a compound represented by the following general formula (II):

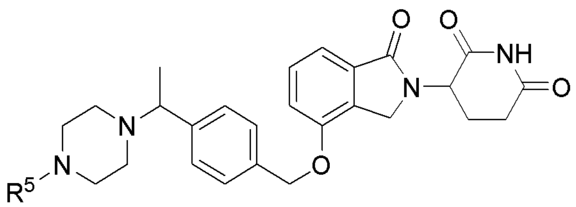

(II)

wherein:
$R^5$ is H, $C_{1-6}$alkyl, $C_{3-6}$cycloalkyl, $-C(O)C_{1-6}$alkyl, $-C(O)C_{3-6}$cycloalkyl, $-S(O)_2C_{1-6}$alkyl, phenyl or 5- to 6-membered heteroaryl containing N, O and/or S, wherein one or more hydrogens of the alkyl, the cycloalkyl, the phenyl and the heteroaryl are optionally substituted with D, halogen, cyano or $C_{1-2}$alkyl.

In a preferable embodiment, $R^5$ is phenyl, pyridinyl or pyrimidinyl, wherein one or two hydrogens of the phenyl, the pyridinyl and the pyrimidinyl are optionally substituted with F or cyano.

The present invention further relates to the following compounds 1-22, or a pharmaceutically acceptable salt, a stable isotope derivative, an isomer thereof, a prodrug thereof, or a mixture thereof:

| Compound No. | Compound structure and name |
|---|---|
| 1. | 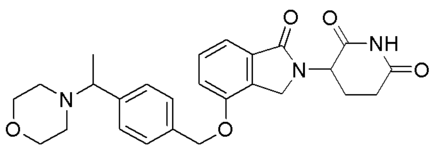 |
| | 3-(4-((4-(1-morpholinoethyl)benzyl)oxy)-1-oxoisoindolin-2-yl)piperidine-2,6-d ione |

(continued)

| Compound No. | Compound structure and name |
|---|---|
| 2. | 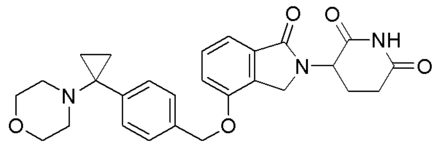 |
| | 3-(4-((4-(1-morpholinocyclopropyl)benzyl)oxy)-1-oxoisoindolin-2-yl)piperidin e-2,6-dione |
| 3. | 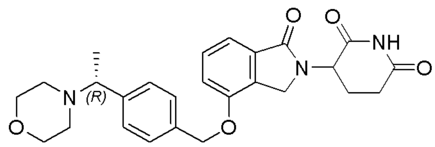 |
| | 3-(4-((4-(*(R)*-1-morpholinoethyl)benzyl)oxy)-1-oxoisoindolin-2-yl)piperidine-2,6-dione |
| 4. | 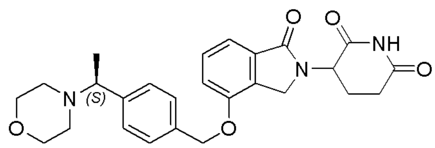 |
| | 3-(4-((4-(*(S)*-1-morpholinoethyl)benzyl)oxy)-1-oxoisoindolin-2-yl)piperidine-2 ,6-dione |
| 5. | 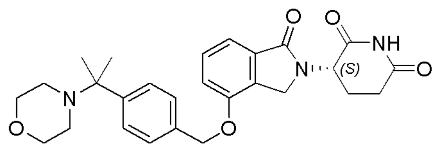 |
| | *(S)*-3-(4-((4-(2-morpholinopropan-2-yl)benzyl)oxy)-1-oxoisoindolin-2-yl)piper idine-2,6-dione |
| 6. | 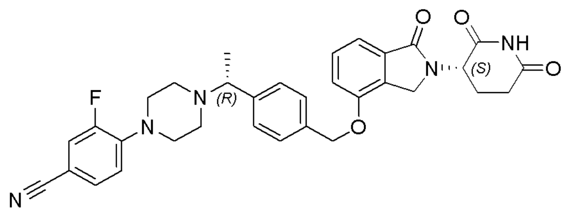 |
| | 4-(4-(*(R)*-1-(4-(((2-(*(S)*-2,6-dioxopiperidin-3-yl)-1-oxoisoindolin-4-yl)oxy)met hyl)phenyl)ethyl)piperazin-1 -yl)-3 -fluorobenzonitrile |
| 7. | 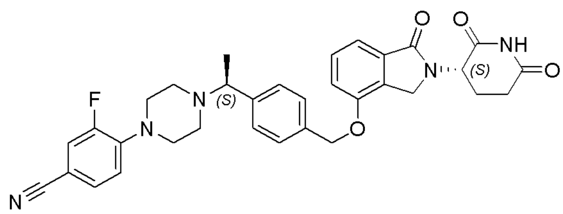 |
| | 4-(4-(*(S)*-1-(4-(((2-(*(S)*-2,6-dioxopiperidin-3-yl)-1-oxoisoindolin-4-yl)oxy)met hyl)phenyl)ethyl)piperazin-1-yl)-3-fluorobenzonitrile |

(continued)

| Compound No. | Compound structure and name |
|---|---|
| 8. | 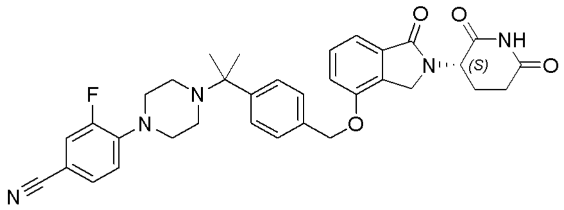 |
| | *(S)*-4-(4-(2-(4-(((2-(2,6-dioxopiperidin-3-yl)-1-oxoisoindolin-4-yl)oxy)methyl) phenyl)prop-2-yl)piperazin-1-yl)-3-fluorobenzonitrile |
| 9. | 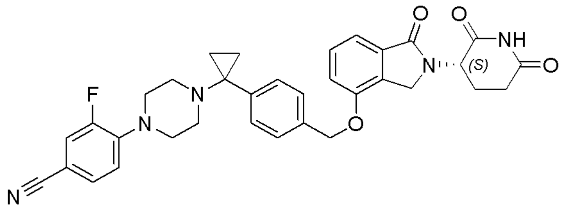 |
| | *(S)*-4-(4-(1-(4-(((2-(2,6-dioxopiperidin-3-yl)-1-oxoisoindolin-4-yl)oxy)methyl) phenyl)cyclopropyl)piperazin-1-yl)-3-fluorobenzonitrile |
| 10. | 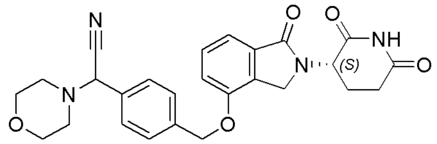 |
| | 2-(4-(((2-(*(S)*-2,6-dioxopiperidin-3-yl)-1-oxoisoindolin-4-yl)oxy)methyl)pheny l)-2-morpholinoacetonitrile |
| 11. | 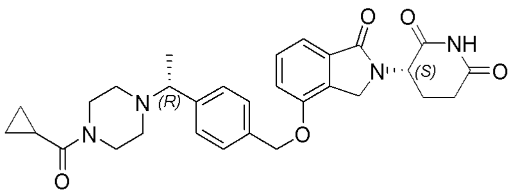 |
| | *(S)*-3-(4-((4-(*(R)*-1-(4-(cyclopropanecarbonyl)piperazin-1-yl)ethyl)benzyl)oxy)-1-oxoisoindolin-2-yl) piperidine-2,6-dione |
| 12. | 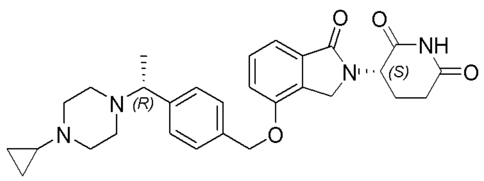 |
| | *(S)*-3-(4-((4-(*(R)*-1-(4-cyclopropylpiperazin-1-yl)ethyl)benzyl)oxy)-1-oxoisoin dolin-2-yl)piperidine-2,6-dione |

(continued)

| Compound No. | Compound structure and name |
|---|---|
| 13. | 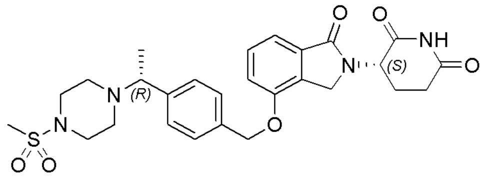 |
| | *(S)*-3-(4-((4-(*(R)*-1-(4-(methylsulfonyl)piperazin-1-yl)ethyl)benzyl)oxy)-1-oxoi soindolin-2-yl)piperidine-2,6-dione |
| 14. | 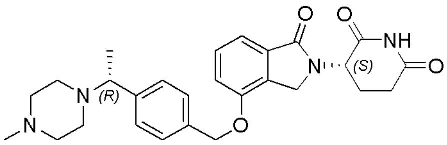 |
| | *(S)*-3-(4-((4-(*(R)*-1-(4-methylpiperazin-1-yl)ethyl)benzyl)oxy)-1-oxoisoindolin-2-yl)piperidine-2,6-dione |
| 15. | 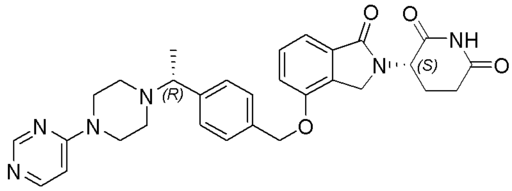 |
| | *(S)*-3-(1-oxo-4-((4-(*(R)*-1-(4-(pyrimidin-4-yl)piperazin-1-yl)ethyl)benzyl)oxy)i soindolin-2-yl)piperidine-2,6-dione |
| 16. | 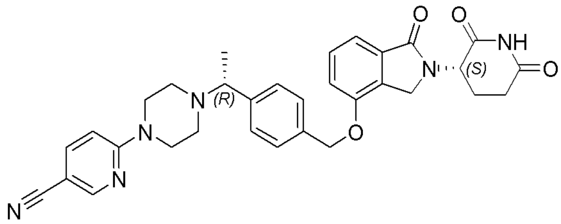 |
| | 6-(4-(*(R)*-1-(4-(((2-(*(S)*-2,6-dioxopiperidin-3-yl)-1-oxoisoindolin-4-yl)oxy)met hyl)phenyl)ethyl)piperazin-1-yl)nicotinonitrile |
| 17. | 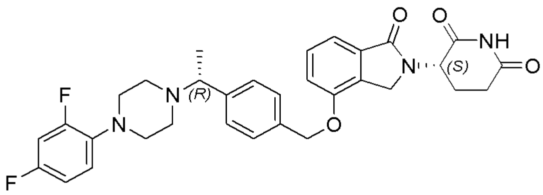 |
| | *(S)*-3-(4-((4-(*(R)*-1-(4-(2,4-difluorophenyl)piperazin-1-yl)ethyl)benzyl)oxy)-1-oxoisoindolin-2-yl)piperidine-2,6-dione |
| 18. | 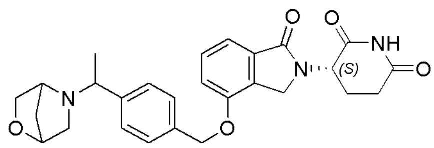 |
| | (3S)-3-(4-((4-(1-(2-oxa-5-azabicyclo[2.2.1]heptan-5-yl)ethyl)benzyl)oxy)-1-ox oisoindolin-2-yl)piperidine-2,6-dione (isomer 1) |

(continued)

| Compound No. | Compound structure and name |
|---|---|
| 19. | 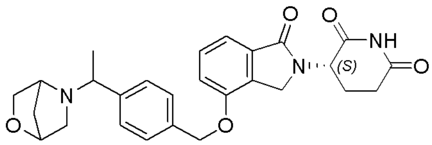 |
| | (3S)-3-(4-((4-(1-(2-oxa-5-azabicyclo[2.2.1]heptan-5-yl)ethyl)benzyl)oxy)-1-ox oisoindolin-2-yl)piperidine-2,6-dione (isomer 2) |
| 20. | 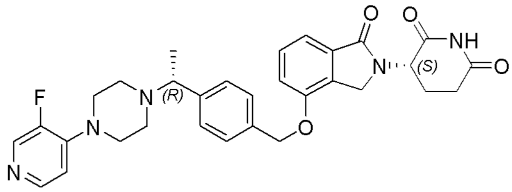 |
| | *(S)*-3-(4-((4-(*(R)*-1-(4-(3-fluoropyridin-4-yl)piperazin-1-yl)ethyl)benzyl)oxy)-1 -oxoisoindolin-2-yl) piperidine-2,6-dione |
| 21. | 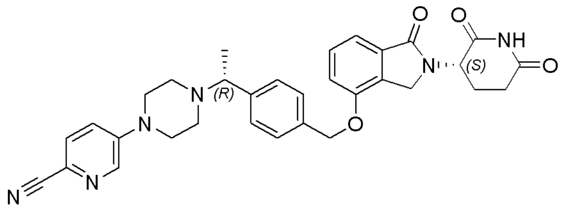 |
| | 5-(4-(*(R)*-1-(4-(((2-(*(S)*-2,6-dioxopiperidin-3-yl)-1-oxoisoindolin-4-yl)oxy)met hyl)phenyl)ethyl)piperazin-1-yl)-2-cyanopyridine |
| 22. | 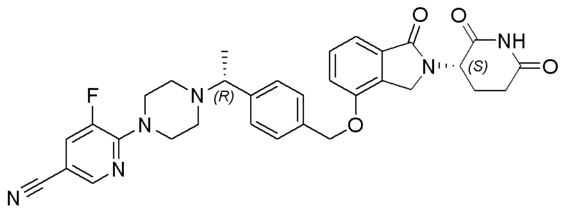 |
| | 6-(4-(*(R)*-1-(4-(((2-(*(S)*-2,6-dioxopiperidin-3-yl)-1-oxoisoindolin-4-yl)oxy)met hyl)phenyl)ethyl)piperazin-1-yl)-5-fluoronicotinonitrile |

The compound of the present invention can effectively inhibit the proliferation of NCI-H929 cells, preferably with an $IC_{50}$ of less than 50 nM, more preferably with an $IC_{50}$ of less than 10 nM. The compound of the present invention has a significant inhibitory effect on the secretion of TNFa in human PBMC cells, preferably with an $IC_{50}$ of less than 20 nM, more preferably with an $IC_{50}$ of less than 10 nM. The compound of the present invention also has a significant stimulation effect on the secretion of IL-2 in human PBMC cells, preferably with an $EC_{50}$ of less than 50 nM, more preferably with an $EC_{50}$ of less than 10 nM.

The present invention also relates to a pharmaceutical composition, which comprises a compound represented by general formula (I) or a pharmaceutically acceptable salt, a stable isotope derivative, an isomer, a prodrug or a mixture thereof and one or more pharmaceutically acceptable carriers or excipients.

One aspect of the present invention provides a compound represented by general formula (I) or a pharmaceutically acceptable salt, a stable isotope derivative, an isomer, a prodrug, or a mixture thereof, or the pharmaceutical composition for use in regulating or inhibiting the activity of E3 ubiquitin ligase, thereby affecting the expression and/or biological function of proteins including but not limited to Aiolos, Ikaros, Helios, CK1α, GSPT1, IL-2, IL-6, TNFα, IFNγ, VEGF and the like.

Another aspect of the present invention provides a method for treating or preventing a correlated disease mediated by Aiolos, Ikaros, Helios, CK1α, GSPT1, IL-2, IL-6, TNFα, IFNγ, VEGF or the like, wherein said method comprises administrating a therapeutically effective amount of the compound represented by general formula (I) or a pharmaceutically acceptable salt, a stable isotope derivative, an isomer, a prodrug, or a mixture thereof, or a pharmaceutical composition containing the compound to a subject in need thereof. Said disease includes, but is not limited to, hematological tumors (such as multiple myeloma, lymphoma, and leukemia), solid tumors (such as lung cancer, prostate cancer, head and neck cancer, breast cancer, ovarian cancer, uterine cancer, pancreatic cancer, colon cancer, rectal cancer, gastric cancer, esophageal cancer, brain cancer, liver cancer, kidney cancer, skin cancer, epithelial cancer, bladder cancer, and neuroblastoma), autoimmune diseases (such as systemic lupus erythematosus, psoriasis, and inflammatory bowel disease), inflammations (such as rheumatoid arthritis), neurodegenerative diseases (such as multiple sclerosis, Alzheimer's disease, and Parkinson's disease), fibrosis (such as pulmonary fibrosis), skin diseases (such as melanoma), eye disease, chronic obstructive pulmonary disease, and the like, especially multiple myeloma, lymphoma, myelodysplastic syndrome, systemic lupus erythematosus, solid tumors, and the like. The present invention also relates to a pharmaceutical composition, which comprises a compound represented by general formula (I) or a pharmaceutically acceptable salt, a stable isotope derivative, an isomer, a prodrug, or a mixture thereof and at least one additional drug, wherein the at least one additional drug can be a small molecule chemotherapeutic agent (such as NSAIDs, steroid anti-inflammatory drugs, kinase targeting drugs, cytotoxic drugs, and DNA damage-related drugs) or a macromolecule immune and/or inflammatory regulators (such as PD-1 antibody, CD20 antibody, CD19 antibody, TNFα antibody, and IL-6 antibody).

According to the present invention, the drug may be in any pharmaceutical dosage form, including but not limited to tablets, capsules, solutions, freeze-dried preparations, and injections.

The pharmaceutical preparations of the present invention may be administered in dosage unit forms containing a predetermined amount of active ingredient per dosage unit. Such units may contain, for example, 0.1 mg to 500 mg, preferably 0.5 mg to 100 mg, of the compound of the present invention depending on the condition to be treated, the method of administration and the age, weight and condition of the patient. Furthermore, pharmaceutical preparations of this type can be prepared using methods known in the field of pharmacy, such as mixing the active ingredient with one or more excipients and/or adjuvants.

The pharmaceutical preparations of the present invention may be adapted for administration by any desired suitable method, such as oral (including buccal or sublingual), rectal, nasal, topical (including buccal, sublingual or transdermal), vaginal or parenteral (including subcutaneous, intramuscular, intravenous or intradermal) administration.

The present invention also provides methods for preparing the compounds. The preparation of the compound represented by general formula (I) of the present invention can be accomplished through the following exemplary methods and examples, but these methods and examples should not be considered as limiting the scope of the present invention in any way. The compounds described in the present invention can also be synthesized by synthetic techniques known to those skilled in the art, or a combination of methods known in the art and methods described in the present invention can be used. The products obtained in each reaction step are obtained by separation techniques known in the art, including but not limited to extraction, filtering, distillation, crystallization, chromatographic separation, and the like. The starting materials and chemical reagents required for the synthesis can be routinely synthesized according to the literatures (available on SciFinder) or purchased.

**Synthetic methods**

The heterocyclic compound represented by general formula (I) of the present invention can be synthesized according to the following route: 1) subjecting "lidomide"-compound A1 and intermediate B with a leaving group X (such as halogen) to a substitution reaction under heating in the catalysis with a base (such as potassium carbonate) to produce the target compound A4, wherein the "lidomide"-compound A1 may be purchased or synthesized according to the method of literature (such as CN107739389). The target compound A4 can also be obtained by firstly subjecting compound A2 with intermediate B to the substitution reaction to produce A3, and then subjecting A3 to the ring closure under heating in the catalysis with an acid (such as TsOH), wherein the compound A2 may be purchased or synthesized according to the method of literature (such as WO2014025978).

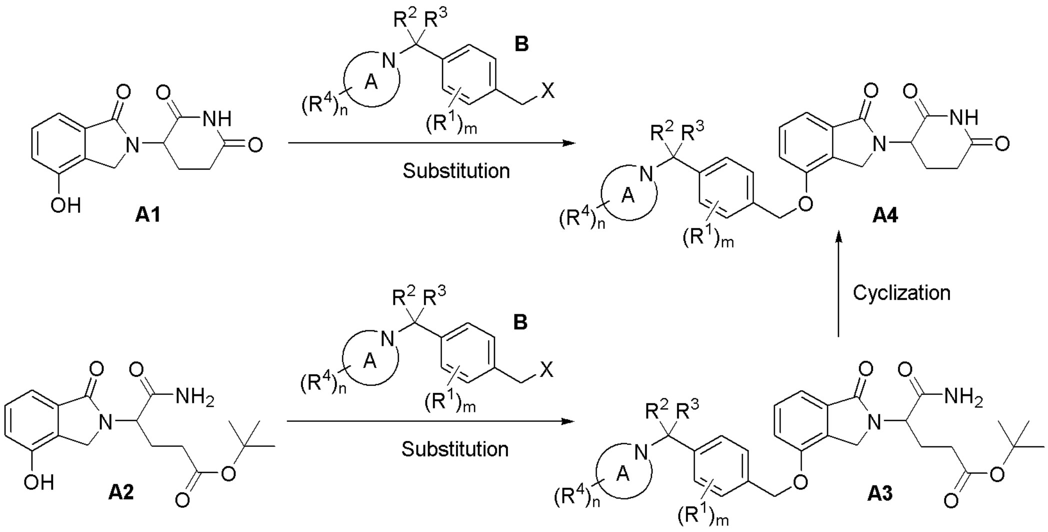


A1 A4 A2 A3

Intermediate B can be synthesized according to the route shown below: 1) subjecting the starting material B1 and the raw material or reagent with two leaving groups X (such as halogen) to cyclization under heating in an alkaline condition (e.g., an organic base DIPEA or an inorganic base potassium carbonate in DMF) to produce an intermediate B2; 2) subjecting a solution of B2 in methanol to a carbonyl insertion reaction under catalysis with a palladium catalyst (such as $PdCl_2$(dppf)) in a carbon monoxide atmosphere to produce an intermediate ester B3; B3 can also be produced by subjecting B4 and the raw material or reagent of NH-containing heterocycle A to a substitution reaction under catalysis of a base (such as potassium carbonate); 3) reducing the ester group in B3 with LAH to produce an intermediate alcohol B5; and 4) further halogenating B5 (for example with $CBr_4/PPh_3$ or $SOCl_2$) to thereby produce the important intermediate B.

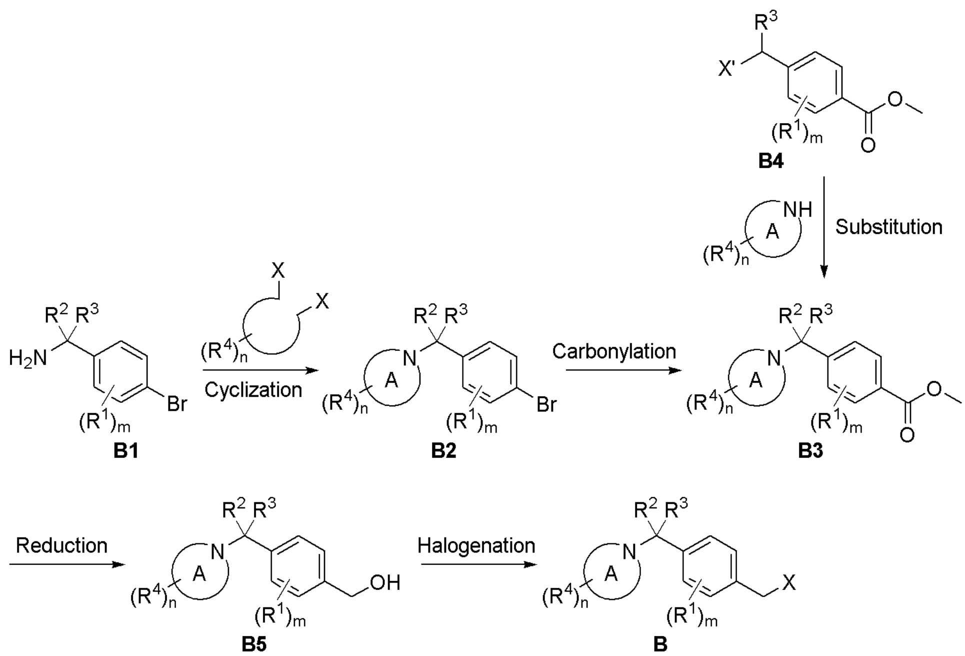


B4 B1 B2 B3 B5 B

## Example

The starting materials of the present invention could be synthesized according to methods known in the art, or could be purchased from chemical companies such as ABCR GmbH&Co. KG, Acros Organics, Aldrich Chemical Company, Accela ChemBio Inc., and Beijing OUHE technology Co. Ltd.

The structures of the compounds of the present invention were determined by nuclear magnetic resonance (NMR) and/or mass spectroscopy (MS). The NMR determination was performed with a Bruker ASCEND-400 nuclear magnetic analyzer, by using the solvent such as deuterated dimethyl sulfoxide (DMSO-d6), deuterated chloroform ($CDCl_3$) or deuterated methanol ($CD_3OD$), using tetramethylsilane (TMS) as the internal standard, and giving chemical

shifts in units of $10^{-6}$ (ppm). The MS determination was performed with an Agilent SQD (ESI) mass spectrometer (Agilent 6120).

The HPLC determination was performed with Agilent 1260 DAD high pressure liquid chromatograph (Poroshell 120 EC-C18, 50x3.0 mm, 2.7 μm chromatographic column) or Waters Arc high pressure liquid chromatograph (Sunfirc C18, 150×4.6 mm, 5 μm chromatographic column).

Unless otherwise specified in the examples, the reaction temperature was room temperature (20 °C to 30°C).

Unless otherwise specified in the examples, the reactions were carried out under an argon atmosphere or a nitrogen atmosphere. Argon atmosphere or nitrogen atmosphere was meant that the reaction bottle was connected to an argon or nitrogen balloon with a volume of about 1 L.

Hydrogen atmosphere was meant that the reaction bottle was connected to a hydrogen balloon with a volume of about 1 L after being vacuumed and then filled with hydrogen (repeatedly 3 times).

A CEM Discover-SP type microwave reactor was used in the microwave reaction.

The reaction progress in the examples was monitored with an LC/MS chromatography (1260/6120) of Agilent, or a thin-layer chromatography (TLC) using a silica gel plate having a thickness of 0.15 to 0.2 mm (Qingdao Haiyang GF254).

The purification of the compound was performed with a column chromatography using 200-300 mesh silica gel from Qingdao Haiyang or a thin-layer chromatography using a GF254 silica gel plate from Qingdao Haiyang having a thickness of 0.4 to 0.5 mm.

The developing solvent system for column chromatography or thin layer chromatography usually included a) dichloromethane and methanol system, b) petroleum ether and ethyl acetate system, or those as shown in the Examples. The volume ratio of solvents was adjusted according to the polarity of the compound, and could also be further adjusted by adding a small amount of triethylamine or other acidic or basic reagents.

The purification of the compound was also performed with a mass spectrometer-guided automatic preparation system (mass spectrometer detector: SQD2) of Waters, and a reversed-phase high-pressure column (XBridge-C18, 19×150 mm, 5 μm) was eluted at a flow rate of 20 mL/min with an appropriate acetonitrile/water (containing 0.1% trifluoroacetic acid or formic acid, or 0.05% ammonia water) gradient according to the polarity of the compound. In a part of examples, 1 N diluted hydrochloric acid could be added after the purification with the automatic preparation system, and then the solvent was removed under a reduced pressure to produce a hydrochloride.

The abbreviation of $PdCl_2$(dppf) refers to [1,1'-bis(diphenylphosphino)ferrocene]palladium(II) dichloride.

The abbreviation of LAH refers to lithium aluminum hydride.

The abbreviation of DIPEA refers to N,N-diisopropylethylamine.

The abbreviation of $TsOH{\cdot}H_2O$ refers to p-toluenesulfonic acid monohydrate.

The abbreviation of THF refers to tetrahydrofuran.

The abbreviation of DMF refers to N,N-dimethylformamide.

**Example 1**

**3-(4-((4-(1-morpholinoethyl)benzyl)oxy)-1-oxoisoindolin-2-yl)piperidine-2,6-dione (Compound 1)**

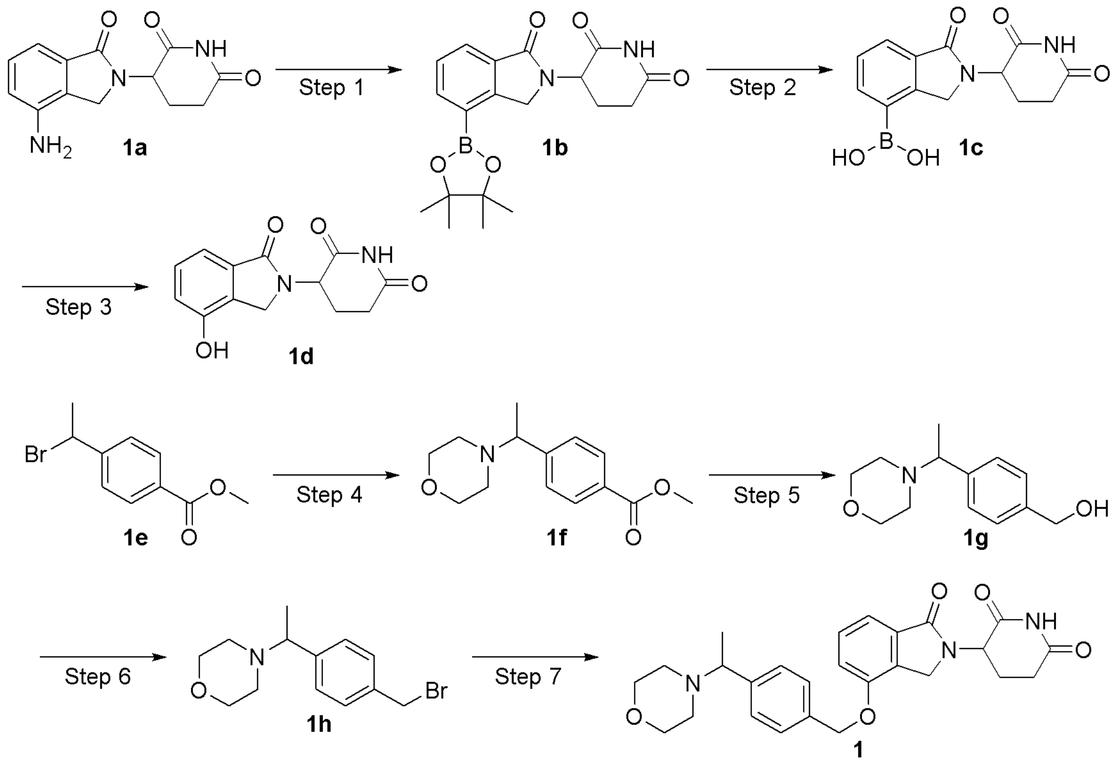


1a 1b 1c 1d 1e 1f 1g 1h 1

Step 1

3-(1-oxo-4-(4,4,5,5-tetramethyl-1,3,2-dioxaborolan-2-yl)isoindolin-2-yl)piperidine-2,6-dione (**1b**)

3-(7-amino-3-oxo-1H-isoindolin-2-yl)piperidine-2,6-dione **1a** (5 g, 19.3 mmol), bis(pinacolato)diboron (7.35 g, 29.0 mmol) and acetonitrile (100 mL) were mixed at room temperature, and then tert-butyl nitrite (2.19 g, 21.3 mmol) was added. The mixture was stirred at room temperature for 18 hours, and then the solvent was removed under a reduced pressure. The residue was purified by a silica gel column chromatography (dichloromethane/methanol=100/1) to produce the target product **1b** (3.9 g, 55%).

MS m/z (ESI): 371 [M+1]

Step 2

(2-(2,6-dioxopiperidin-3-yl)-1-oxoisoindolin-4-yl)boronic acid (**1c**)

**1b** (3.9 g, 10.5 mmol), THF (100 mL) and water (20 mL) were mixed at room temperature, and then sodium periodate (6.7 g, 31.6 mmol) was slowly added. The mixture was stirred at room temperature for 2 hours, and then a diluted hydrochloric acid (1 N, 7.3 mL, 7.3 mmol) was added. The mixture was stirred at room temperature for 18 hours, and the solvent was removed under a reduced pressure. The residue was dissolved in dichloromethane (20 mL) and water (20 mL). The mixture was stirred at room temperature for 18 hours, and filtered to produce the target product **1c** (2.3 g, 76%). MS m/z (ESI): 289 [M+1]

Step 3

3-(4-hydroxy-1-oxoisoindolin-2-yl)piperidine-2,6-dione (**1d**)

To a solution of **1c** (1 g, 3.5 mmol) in dimethyl sulfoxide (20 mL) was added an aqueous hydrogen peroxide solution (2.76 mL, 27 mmol) under the nitrogen gas atmosphere. The mixture was stirred at room temperature for 18 hours, and then water (30 mL) was added. The resulting mixture was extracted with ethyl acetate (3×50 mL). The combined organic phases were washed with a saturated saline solution (3×50 mL), and then dried over anhydrous sodium sulfate. After filtering, the filtrate was concentrated to dryness under a reduced pressure. The residue was purified by a silica gel column chromatography (dichloromethane/methanol=100/1 to 85/15) to produce the target product **1d**

(110 mg, 12%).

MS m/z (ESI): 261 [M+1]

Step 4

Methyl 4-(1-morpholinoethyl)benzoate (**1f**)

Methyl 4-(1-bromoethyl)benzoate **1e** (242 mg, 1 mmol), morpholine (87 mg, 1 mmol), potassium carbonate (151.8 mg, 1.1 mmol) and acetonitrile (1 mL) were mixed at room temperature and stirred for 18 hours, and then water (3 mL) was added. The mixture was extracted with ethyl acetate (3×3 mL). The combined organic phases were washed with water (3×2 mL), and then dried over anhydrous sodium sulfate. After filtering, the filtrate was concentrated to dryness under a reduced pressure to produce the target product **1f** (280 mg, crude product). The product was not further purified and directly used in the next step reaction.

MS m/z (ESI): 250 [M+1]

Step 5

(4-(1-morpholinoethyl)phenyl)methanol (**1g**)

**1f** (280 mg, crude product) was dissolved in THF (2 mL) and cooled to 0°C. Then LAH (128 mg, 3.37 mmol) was added in a nitrogen gas atmosphere. The mixture was stirred at 0°C for 1 hour, and then stirred at room temperature for 3 hours. To the mixture was added water (2 mL), and then the resulting mixture was extracted with ethyl acetate (3×3 mL). The combined organic phases were washed with water (3×2 mL), and then dried over anhydrous sodium sulfate. After filtering, the filtrate was concentrated to dryness under a reduced pressure. The residue was purified by a silica gel column chromatography (petroleum ether/ethyl acetate=100/1 to 1/1) to produce the target product **1g** (160 mg, 73%).

MS m/z (ESI): 222 [M+1]

Step 6

4-(1-(4-(bromomethyl)phenyl)ethyl)morpholine (**1h**)

**1g** (80 mg, 0.36 mmol), tetrabromomethane (143 mg, 0.43 mmol) and dichloromethane (5 mL) were mixed, and then triphenylphosphine (114 mg, 0.43 mmol) was added in a nitrogen gas atmosphere. The mixture was stirred at room temperature for 48 hours, and the solvent was removed under a reduced pressure. The residue was purified by a silica gel column chromatography (petroleum ether/ethyl acetate=100/1 to 7/3) to produce the target product **1h** (20 mg, 20%).

MS m/z (ESI): 284 [M+1]

Step 7

3-(4-((4-(1-morpholinoethyl)benzyl)oxy)-1-oxoisoindolin-2-yl)piperidine-2,6-dione (1) **1h** (20 mg, 0.07 mmol), **1d** (18.4 mg, 0.07 mmol), potassium iodide (17.6 mg, 0.11 mmol), potassium carbonate (14.6 mg, 0.11 mmol) and acetonitrile (1 mL) were mixed at room temperature. The mixture was heated to 80°C, stirred for 18 hours, and cooled to room temperature. The solvent was removed under a reduced pressure. The residue was purified with a reversed-phase preparative high performance liquid chromatography to produce the target product 1 (6.1 mg, solid, 19%).

MS m/z (ESI): 464 [M+1]

$^1$H NMR (400 MHz, $CD_3OD$) δ 7.41 - 7.36 (m, 3H), 7.32 - 7.27 (m, 3H), 7.19 (d, *J*=8.0 Hz, 1H), 5.14 (s, 2H), 5.06 - 5.02 (m, 1H), 4.35 (q, *J*=17.4 Hz, 3H), 3.63 - 3.57 (m, 4H), 2.80 - 2.75 (m, 1H), 2.72 - 2.67 (m, 1H), 2.48 - 2.34 (m, 4H), 2.12 - 2.01 (m, 2H), 1.36 (d, *J*=6.7 Hz, 3H).

**Example 2**

**3-(4-((4-(1-morpholinocyclopropyl)benzyl)oxy)-1-oxoisoindolin-2-yl)piperidine-2,6-dione (Compound 2)**

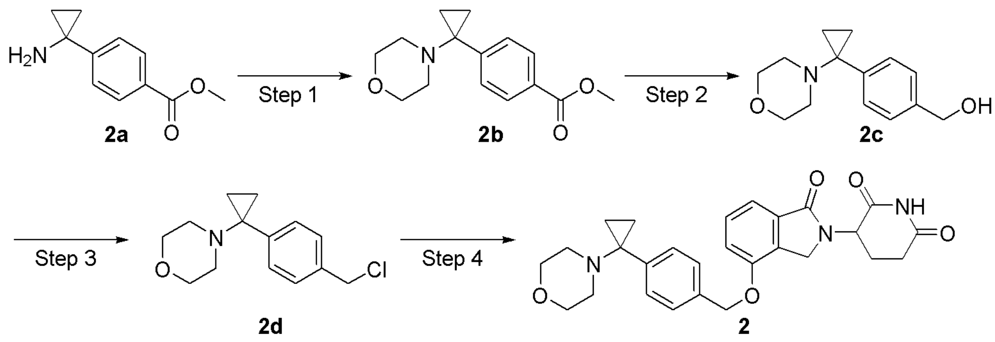


2a 2b 2c 2d 2

Step 1

Methyl 4-(1-morpholinocyclopropyl)benzoate (**2b**)

Methyl 4-(1-aminocyclopropyl)benzoate **2a** (191 mg, 1 mmol), 1-bromo-2-(2-bromoethoxy)ethane (696 mg, 3 mmol), DIPEA (645 mg, 5 mmol) and DMF (3 mL) were mixed at room temperature. The mixture was heated to 110°C, stirred for 18 hours, and cooled to room temperature. Water (10 mL) was added. Then the resulting mixture was extracted with ethyl acetate (3×10 mL). The combined organic phases were washed with water (3×5 mL), and then dried over anhydrous sodium sulfate. After filtering, the filtrate was concentrated to dryness under a reduced pressure. The residue was purified by a silica gel column chromatography (petroleum ether/ethyl acetate=100/1 to 19/1) to produce the target product **2b** (150 mg, 57%).

MS m/z (ESI): 262 [M+1]

Step 2

(4-(1-morpholinocyclopropyl)phenyl)methanol (**2c**)

To a solution of **2b** (150 mg, 0.57 mmol) in THF (5 mL) was added LAH (43.6 mg, 1.15 mmol) at 0°C. The mixture was stirred at room temperature for 2 hours, and water was added (2 mL). Then the resulting mixture was extracted with ethyl acetate (3×10 mL). The combined organic phases were washed with water (3×5 mL), and then dried over anhydrous sodium sulfate. After filtering, the filtrate was concentrated to dryness under a reduced pressure to produce the target product **2c** (110 mg, 84%). The product was not further purified and directly used in the next step reaction.

MS m/z (ESI): 234 [M+1]

Step 3

4-(1-(4-(chloromethyl)phenyl)cyclopropyl)morpholine (**2d**)

To a solution of **2c** (110 mg, 0.47 mmol) in dichloromethane (5 mL) was added thionyl chloride (84 mg, 0.71 mmol) at room temperature. The mixture was stirred at room temperature for 2 hours, and the solvent was removed under a reduced pressure to produce the target product **2d** (100 mg, 20%). The product was not further purified and directly used in the next step reaction.

MS m/z (ESI): 252 [M+1]

Step 4

3-(4-((4-(1-morpholinocyclopropyl)benzyl)oxy)-1-oxoisoindolin-2-yl)piperidine-2,6-di one (**2**)

**2d** (100 mg, 0.4 mmol), **1d** (52 mg, 0.2 mmol), potassium iodide (66 mg, 0.4 mmol), potassium carbonate (55 mg, 0.4mmol) and acetonitrile (5 mL) were mixed at room temperature. The mixture was heated to 100°C, stirred for 18 hours, cooled to room temperature and filtered. The filtrate was concentrated to dryness under a reduced pressure. The residue was purified with a reversed-phase preparative high performance liquid chromatography to produce the target product **2** (5.2 mg, solid, 5.5%).

MS m/z (ESI): 476 [M+1]

$^1$H NMR (400 MHz, $CD_3OD$) δ 7.48 (t, *J*=7.3 Hz, 3H), 7.41 (d, *J*=7.3 Hz, 3H), 7.29 (d, *J*=7.9 Hz, 1H), 5.25 (s,

2H), 5.14 (dd, *J*=13.3, 5.1 Hz, 1H), 4.45 (q, *J*=17.3 Hz, 2H), 3.73 - 7.55 (m, 4H), 2.91 - 2.85 (m, 1H), 2.81 - 2.64 (m, 4H), 2.50 (dd, *J*=13.2, 4.7 Hz, 1H), 2.20 - 2.13 (m, 1H), 1.31 - 1.23 (m, 1H), 1.15 - 1.03 (m, 2H), 0.98 - 0.88 (m, 2H).

**Example 3**

**3-(4-((4-(*(R)*-1-morpholinoethyl)benzyl)oxy)-1-oxoisoindolin-2-yl)piperidine-2,6-di one (Compound 3)**

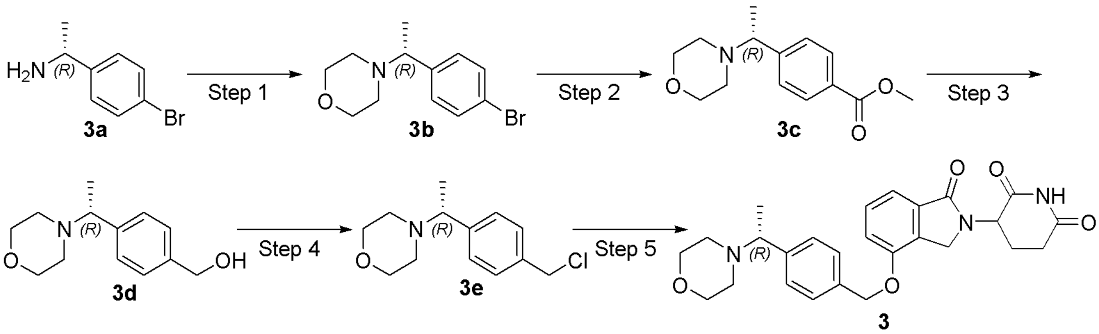


Step 1

*(R)*-4-(1-(4-bromophenyl)ethyl)morpholine (**3b**)

*(R)*-1-(4-bromophenyl)ethylamine **3a** (2 g, 10 mmol) was dissolved in DMF (40 mL), and then potassium carbonate (4.15 g, 30 mmol) and 1-bromo-2-(2-bromoethoxy)ethane (2.58 g, 11.1 mmol) were added. The mixture was stirred at room temperature for 3 hours, and then heated to 80°C and stirred for 3 hours. The mixture was cooled to room temperature, and 1-bromo-2-(2-bromoethoxy)ethane (258 mg, 1.11 mmol) was supplemented. The mixture was heated to 80°C and stirred for 2 hours. The mixture was cooled to room temperature, and diluted with water (250 mL), and then extracted with ethyl acetate (2×150 mL). The combined organic phases were successively washed with a saturated sodium bicarbonate solution (2×150 mL) and a saturated saline solution (150 mL), and then dried over anhydrous sodium sulfate. After filtering, the filtrate was concentrated to dryness under a reduced pressure. The residue was purified by a silica gel column chromatography (dichloromethane/methanol=100/0 to 96/4) to produce the target product **3b** (2.14 g, 79%).

MS m/z (ESI): 270 [M+1]

$^1$H NMR (400 MHz, $CDCl_3$) δ 7.44 (d, *J*=8.3 Hz, 2H), 7.21 (d, *J*=8.0 Hz, 2H), 3.77 -3.60 (m, 4H), 3.27 (d, *J*=4.6 Hz, 1H), 2.46 (s, 2H), 2.36 (s, 2H), 1.30 (t, *J*=9.0 Hz, 3H).

Step 2

*(R)*-methyl 4-(1-morpholinoethyl)benzoate (**3c**)

**3b** (2.14 g, 7.92 mmol) was dissolved in methanol (80 mL), and triethylamine (4 g, 39.6 mmol) was added. The mixture was cooled to -40°C, and bubbled with a carbon monoxide gas for 30 minutes. Then $PdCl_2$(dppf) (1.16 g, 1.58 mmol) was added. The mixture was heated to 100°C in a sealed tube and stirred for 20 hours. The mixture was cooled to room temperature and filtered. The filtrate was concentrated to dryness under a reduced pressure. The residue was purified by a silica gel column chromatography (petroleum ether/ethyl acetate=100/0 to 3/7) to produce the target product **3c** (1.7 g, 86%).

MS m/z (ESI): 250 [M+1]

$^1$H NMR (400 MHz, $CDCl_3$) δ 7.99 (d, *J*=4.7 Hz, 2H), 7.41 (s, 2H), 3.91 (s, 3H), 3.69 (s, 4H), 3.36 (s, 1H), 2.49 (s, 2H), 2.35 (s, 2H), 1.34 (s, 3H).

Step 3

*(R)*-(4-(1-morpholinoethyl)phenyl)methanol (**3d**)

**3c** (1.7 g, 6.82 mmol) was dissolved in anhydrous THF (40 mL) and cooled to 0°C. Then LAH (600 mg, 15.8

mmol) was added. The mixture was stirred at room temperature for 16 hours, cooled to 0°C, and then quenched with a NaOH solution (1 N, 8 mL). After filtering, the filtrate was dried over anhydrous sodium sulfate, and then filtered again. The resulting filtrate was concentrated to dryness under a reduced pressure. The residue was purified by a silica gel column chromatography (dichloromethane/methanol=100/0 to 98/2) to produce the target product **3d** (1.45 g, 96%).

MS m/z (ESI): 222 [M+1]

$^1$H NMR (400 MHz, $CDCl_3$) δ 7.33 (s, 4H), 4.68 (s, 2H), 3.70 (s, 4H), 3.33 (s, 1H), 2.50 (s, 2H), 2.39 (s, 2H), 1.72 (s, 1H), 1.37 (s, 3H).

Step 4

*(R)*-4-(1-(4-(chloromethyl)phenyl)ethyl)morpholine (**3e**)

**3d** (98 mg, 0.44 mmol) was dissolved in dichloromethane (10 mL). Then thionyl chloride (1 mL) was added. The mixture was stirred at room temperature for 2 hours, and concentrated to dryness to produce the target product **3e** (120 mg, crude product). The product was not further purified and directly used in the next step reaction.

MS m/z (ESI): 240 [M+1]

Step 5

3-(4-((4-(*(R)*-1-morpholinoethyl)benzyl)oxy)-1-oxoisoindolin-2-yl)piperidine-2,6-dione formate (**3**)

**3e** (120 mg, crude product), potassium carbonate (182 mg, 1.32 mmol), **1d** (92 mg, 0.35 mmol), potassium iodide (73 mg, 0.44 mmol) and acetonitrile (10 mL) were mixed, and heated to 80°C and stirred for 3 hours. The mixture was cooled to room temperature, and the solvent was removed under a reduced pressure. The residue was diluted with water (20 mL), and then extracted with ethyl acetate (2×20 mL). The combined organic phases were dried over anhydrous sodium sulfate, and filtered. The resulting filtrate was concentrated to dryness under a reduced pressure. The residue was purified with a reversed-phase preparative high performance liquid chromatography to produce the target product **3** (34.5 mg, solid, 19%).

MS m/z (ESI): 464 [M+1]

$^1$H NMR (400 MHz, $CD_3OD$) δ 8.29 (s, 1H), 7.48 (dd, *J*=7.9, 6.3 Hz, 3H), 7.40 (dd, *J*=7.7, 3.0 Hz, 3H), 7.28 (d, *J*=8.0 Hz, 1H), 5.23 (s, 2H), 5.14 (dd, *J*=13.3, 5.2 Hz, 1H), 4.45 (q, *J*=17.4 Hz, 2H), 3.71 (t, *J*=4.7 Hz, 4H), 3.62 (dd, *J*=13.5, 6.7 Hz, 1H), 2.95 - 2.84 (m, 1H), 2.80 - 2.66 (m, 3H), 2.58 - 2.44 (m, 3H), 2.16 (dtd, *J*=12.8, 5.2, 2.3 Hz, 1H), 1.46 (d, *J*=6.8 Hz, 3H).

Compound 4 was prepared according to the experimental procedures from step 1 to step 5 for compound 3, but in step 1, *(S)*-1-(4-bromophenyl)ethylamine was used to replace 3a.

| Compound No. | Compound structure | Compound in replacement of 3**a** | MS m/z (ESI) |
|---|---|---|---|
| **4** | 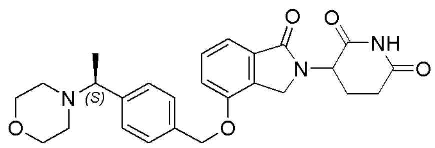 | 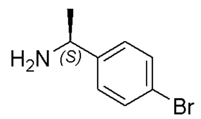 | 464 |

The NMR data of Compound 4 was as follows:

| Compound | $^1$H NMR |
|---|---|
| 3-(4-((4-(*(S)*-1-morph olinoethyl) benzyl)oxy )-1-oxoisoindolin-2-yl ) piperidine-2,6-dione (**4**) | $^1$H NMR (400 MHz, $CD_3OD$) δ 8.30 (s, 1H), 7.48 (dd, *J*=7.8, 6.1 Hz, 3H), 7.40 (dd, *J*=7.8, 3.3 Hz, 3H), 7.29 (d, *J*=8.0 Hz, 1H), 5.24 (s, 2H), 5.14 (dd, *J*=13.3, 5.1 Hz, 1H), 4.45 (d, *J*=12.3 Hz, 2H), 3.71 (t, *J*=4.7 Hz, 4H), 3.65 - 3.59 (m, 1H), 2.94 - 2.85 (m, 1H), 2.82 - 2.65 (m, 3H), 2.53 (d, *J*=11.1 Hz, 3H), 2.16 (ddd, *J*=10.3, 5.2, 2.9 Hz, 1H), 1.46 (d, *J*=6.8 Hz, 3H). |

**Example 4**

***(S)*-3-(4-((4-(2-morpholinopropan-2-yl)benzyl)oxy)-1-oxoisoindolin-2-yl)piperidine-2,6-dione (Compound 5)**

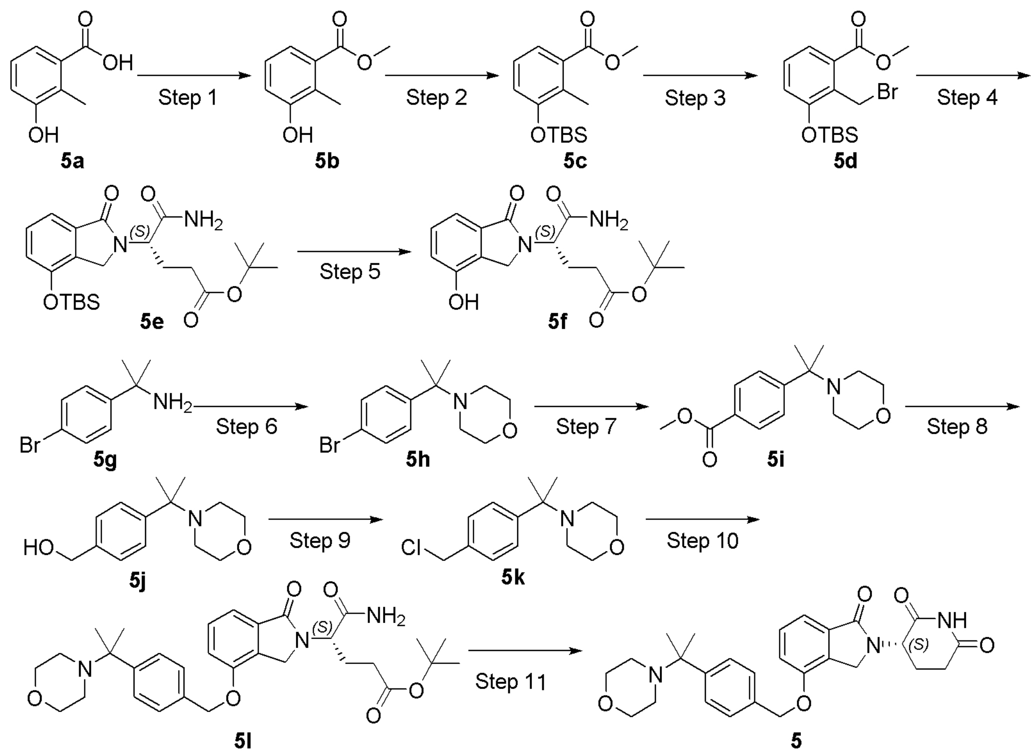


Step 1

Methyl 3-hydroxy-2-methylbenzoate (**5b**)

3-hydroxy-2-methylbenzoic acid **5a** (5 g, 33 mmol) was dissolved in methanol (50 mL). Then a concentrated sulfuric acid (980 mg, 9.9 mmol) was added. The mixture was heated to 70°C, stirred for 20 hours, then cooled to room temperature, and concentrated to about 10 mL. Then the residue was slowly added to cold water (100 mL). Then the resulting mixture was adjusted with a saturated sodium bicarbonate solution to the pH of 4, stirred for 20 minutes, and filtered to collect the precipitate to produce the target product **5b** (4.48 g, 82%).
MS m/z (ESI): 167[M+1]
$^1$H NMR (400 MHz, DMSO-$d_6$) δ 9.67 (s, 1H), 7.17 (d, *J*=7.7 Hz, 1H), 7.08 (t, *J*=7.8 Hz, 1H), 6.99 (d, *J*=7.9 Hz, 1H), 3.79 (s, 3H), 2.27 (s, 3H).

Step 2

Methyl 3-((tert-butyldimethylsilyl)oxy)-2-methylbenzoate (**5c**)

**5b** (4.48 g, 27 mmol) was dissolved in DMF (25 mL). The mixture was cooled to 0°C, and imidazole (4.59 g, 67 mmol) and tert-butyldimethylchlorosilane (4.47 g, 30 mmol) were successively added. The mixture was warmed up to room temperature, stirred for 16 hours, and then diluted with water (125 mL). The resulting mixture was extracted with ethyl acetate (2×100 mL). The combined organic phases were washed with water (3×100 mL), and then dried over anhydrous sodium sulfate. After filtering, the filtrate was concentrated to dryness under a reduced pressure. The residue was purified by a silica gel column chromatography (petroleum ether/ethyl acetate=100/0 to 93/7) to produce the target product **5c** (7.57g, 100%).
MS m/z (ESI): 281[M+1]
$^1$H NMR (400 MHz, $CDCl_3$) δ 7.21 (dd, *J*=7.8, 1.0 Hz, 1H), 6.87 (t, *J*=7.9 Hz, 1H), 6.71 (dd, *J*=8.0, 0.9 Hz, 1H), 3.66 (s, 3H), 2.20 (s, 3H), 0.83 - 0.77 (m, 9H), 0.02 -0.04 (m, 6H).

Step 3

Methyl 2-(bromomethyl)-3-((tert-butyldimethylsilyl)oxy)benzoate (**5d**)

**5c** (561 mg, 2 mmol) was dissolved in tetrachloromethane (30 mL). N-bromosuccinimide (374 mg, 2.1 mmol) and dibenzoyl peroxide (41 mg, 0.2 mmol) were added. The mixture was heated to 80°C, stirred for 3 hours, then cooled to room temperature, and filtered. The filtrate was concentrated to dryness under a reduced pressure. The residue was purified by a silica gel column chromatography (petroleum ether/ethyl acetate=100/0 to 97/3) to produce the target product **5d** (630 mg, 88%).

$^1$H NMR (400 MHz, $CDCl_3$) δ 7.52 (dd, *J*=7.8, 1.2 Hz, 1H), 7.23 (t, *J*=8.0 Hz, 1H), 7.00 (dd, *J*=8.2, 1.1 Hz, 1H), 5.02 (s, 2H), 3.93 (s, 3H), 1.10 - 1.03 (m, 9H), 0.33 - 0.26 (m, 6H).

Step 4

Tert-butyl *(S)*-5-amino-4-(4-((tert-butyldimethylsilyl)oxy)-1-oxoisoindolin-2-yl)-5-oxopentanoate (**5e**)

**5d** (630 mg, 1.75 mmol) and tert-butyl (S)-4,5-diamino-5-oxopentanoate hydrochloride (460 mg, 1.93 mmol) was dissolved in acetonitrile (5 mL). Then DIPEA (566 mg, 4.38 mmol) was added. The mixture was heated to 50°C, stirred for 18 hours, and cooled to room temperature. The solvent was removed under a reduced pressure. The residue was purified by a silica gel column chromatography (petroleum ether/ethyl acetate=44/56 to 0/100) to produce the target product **5e** (545 mg, 69%).

MS m/z (ESI): 449[M+1]

$^1$H NMR (400 MHz, $CDCl_3$) δ 7.47 - 7.43 (m, 1H), 7.38 - 7.32 (m, 1H), 6.98 - 6.94 (m, 1H), 6.33 (s, 1H), 5.36 - 5.31 (m, 1H), 4.91 - 4.85 (m, 1H), 4.43 - 4.30 (m, 2H), 2.48 - 2.09 (m, 4H), 1.42 (s, 9H), 1.03 - 0.98 (m, 9H), 0.28 - 0.22 (m, 6H).

Step 5

tert-butyl *(S)*-5-amino-4-(4-hydroxy-1-oxoisoindolin-2-yl)-5-oxopentanoate (**5f**)

**5e** (545 mg, 1.21 mmol) was dissolved in THF (3 mL) at room temperature, and a solution of tetrabutylammonium fluoride in THF (1 M, 1.5 mL, 1.5 mmol) was added. The mixture was stirred for 30 minutes, diluted with water (20 mL), and then extracted with ethyl acetate (3×20 mL). The combined organic phases were dried over anhydrous sodium sulfate. After filtering, the filtrate was concentrated to dryness under a reduced pressure. The residue was purified by a silica gel column chromatography (petroleum ether/ethyl acetate=1/1 to 0/100) to produce the target product **5f** (270 mg, 67%).

MS m/z (ESI): 335[M+1]

Step 6

4-(2-(4-bromophenyl)prop-2-yl)morpholine (**5h**)

2-(4-bromophenyl)propyl-2-amine **5g** (214 mg, 1 mmol) was dissolved in DMF (4 mL), and potassium carbonate (415 mg, 3 mmol) and 1-bromo-2-(2-bromoethoxy)ethane (278 mg, 1.2 mmol) were added. The mixture was heated to 80°C, stirred for 20 hours, cooled to room temperature, and diluted with water (80 mL). The mixture was extracted with ethyl acetate (3×40 mL). The combined organic phases were successively washed with water (2×40 mL) and brine (40 mL), and then dried over anhydrous sodium sulfate. After filtering, the filtrate was concentrated to dryness under a reduced pressure. The residue was purified by a silica gel column chromatography (petroleum ether/ethyl acetate=100/0 to 3/2) to produce the target product **5h** (207 mg, 73%).

MS m/z (ESI): 284 [M+1]

$^1$H NMR (400 MHz, $CDCl_3$)δ7.42 (s, 4H), 3.73 - 3.61 (m, 4H), 2.50 - 2.41 (m, 4H), 1.31 (s, 6H).

Step 7

Methyl 4-(2-morpholinopropan-2-yl)benzoate (**5i**)

**5h** (207 mg, 0.73 mmol) was dissolved in methanol (20 mL). Then triethylamine (369 mg, 3.64 mmol) was added. The mixture was cooled to -40°C, and bubbled with a carbon monoxide gas for 20 minutes. Then $PdCl_2$(dppf)

(107 mg, 0.15 mmol) was added. The mixture was heated to 100°C in a sealed tube, stirred for 20 hours, cooled to room temperature, and filtered through Celite. The filtrate was concentrated to dryness under a reduced pressure. The residue was purified by a silica gel column chromatography (petroleum ether/ethyl acetate=100/0 to 1/1) to produce the target product **5i** (189 mg, 98%).

MS m/z (ESI): 264 [M+1]

$^1$H NMR (400 MHz, $CDCl_3$)δ7.97 (d, *J*=8.5 Hz, 2H), 7.62 (d, *J*=8.5 Hz, 2H), 3.91 (s, 3H), 3.71 - 3.63 (m, 4H), 2.50 - 2.41 (m, 4H), 1.35 (s, 6H).

Step 8

(4-(2-morpholinopropan-2-yl)phenyl)methanol (**5j**)

**5i** (189 mg, 0.72 mmol) was dissolved in THF (20 mL) and cooled to 0°C. Then LAH (35 mg, 1.44 mmol) was added. The mixture was stirred at room temperature for 2 hours, then quenched with a NaOH solution (1 N, 0.2 mL) and filtered. The filtrate was dried over anhydrous sodium sulfate and filtered. The resulting filtrate was concentrated to dryness under a reduced pressure. The residue was purified by a silica gel column chromatography (petroleum ether/ethyl acetate=1/1 to 3/7) to produce the target product **5j** (77 mg, 46%).

MS m/z (ESI): 236 [M+1]

Step 9

4-(2-(4-(chloromethyl)phenyl)prop-2-yl)morpholine (**5k**)

**5j** (77 mg, 0.33 mmol) was dissolved in dichloromethane (2 mL). Then thionyl chloride (0.5 mL) was added. The mixture was stirred at room temperature for 16 hours, and then concentrated to dryness to produce the target product **5k** (solid, crude product). The product was not further purified and directly used in the next step reaction.

MS m/z (ESI): 254 [M+1]

Step 10

tert-butyl *(S)*-5-amino-4-(4-((4-(2-morpholinopropan-2-yl)benzyl)oxy)-1-oxoisoindolin-2-yl)-5-o xopentanoate (**5l**)

**5k** (crude product, about 0.33 mmol) was dissolved in DMF (2 mL). Then potassium carbonate (138 mg, 1 mmol) and **5f** (110 mg, 0.33 mmol) were added. The mixture was heated to 50°C and stirred for 24 hours. The mixture was cooled to room temperature, then diluted with water (20 mL), and then extracted with ethyl acetate (3×20 mL). The combined organic phases were washed with water (3×20 mL), and then dried over anhydrous sodium sulfate. After filtering, the filtrate was concentrated to dryness under a reduced pressure. The residue was purified with a silica gel column chromatography (dichloromethane/methanol=100/0 to 19/1) to produce the target product **5l** (163 mg, two steps: 90%).

MS m/z (ESI): 552 [M+1]

Step 11

*(S)* -3 -(4-((4-(2-morpholinopropan-2-yl)benzyl)oxy)-1 -oxoisoindolin-2-yl)piperidine-2,6 -dione formate

**5l** (163 mg, 0.3 mmol) was dissolved in acetonitrile (2 mL), and TsOH·$H_2O$ (56 mg, 0.3 mmol) was added. The mixture was heated to 90°C, stirred for 18 hours, and cooled to room temperature. TsOH·$H_2O$ (56 mg, 0.3 mmol) was added again. Then the mixture was heated to 90°C and stirred for 2 hours. After cooling the mixture to room temperature, a saturated sodium bicarbonate solution (20 mL) was added. Then the resulting mixture was extracted with ethyl acetate (3×20 mL). The combined organic phases were dried over anhydrous sodium sulfate, and filtered. The filtrate was concentrated to dryness under a reduced pressure. The residue was purified with a reversed-phase preparative high performance liquid chromatography to produce the target product **5** (37 mg, solid, 26%).

MS m/z (ESI): 478 [M+1]

$^1$H NMR (400 MHz, $CD_3OD$) δ 8.26 (s, 1H), 7.62 (d, *J*=8.4 Hz, 2H), 7.52 - 7.45 (m, 3H), 7.42 (d, *J*=7.0 Hz, 1H), 7.31 (d, *J*=7.6 Hz, 1H), 5.25 (s, 2H), 5.16 (dd, *J*=13.3, 5.2 Hz, 1H), 4.47 (q, *J*=17.3 Hz, 2H), 3.75 - 3.67 (m, 4H), 2.96-2.87 (m, 1H), 2.82-2.76 (m, 1H), 2.69 - 2.61 (m, 4H), 2.57-2.46 (m, 1H), 2.21-2.15 (m, 1H), 1.50 (s, 6H).

**Example 5**

**4-(4-(*(R)*-1-(4-(((2-(*(S)*-2,6-dioxopiperidin-3-yl)-1-oxoisoindolin-4-yl)oxy)methyl)ph enyl)ethyl)piperazin-1-yl)-3-fluorobenzonitrile (Compound 6)**

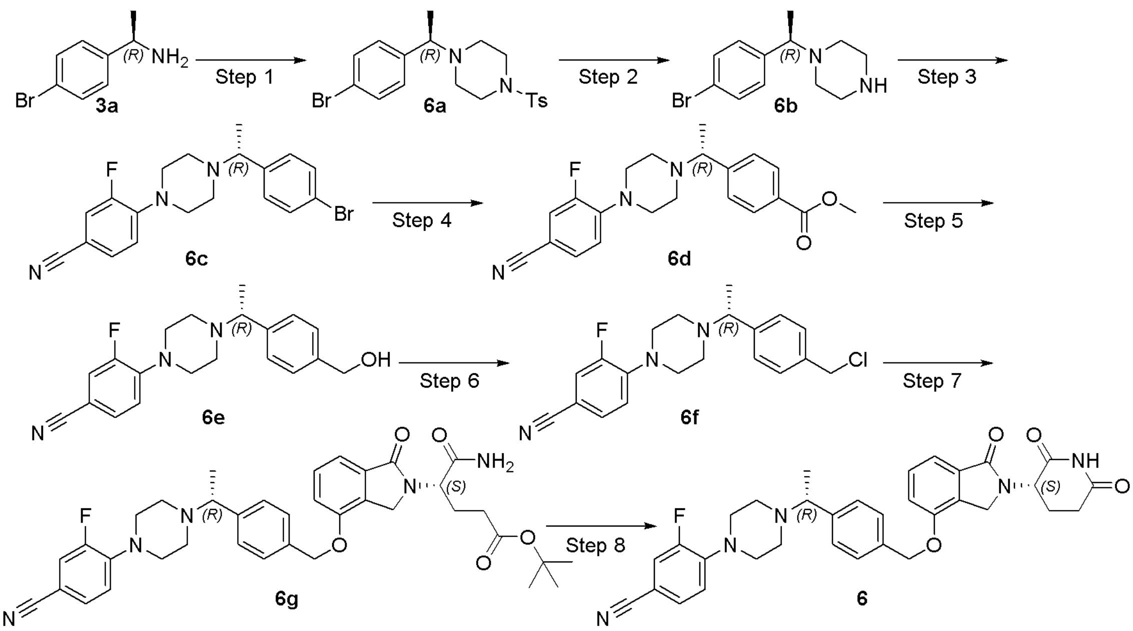


Step 1

*(R)*-1-(1-(4-bromophenyl)ethyl)-4-tosylpiperazine (**6a**)

*(R)*-1-(4-bromophenyl)ethylamine **3a** (2 g, 10 mmol), N,N-bis(2-chloroethyl)-4-methylbenzene sulfonamide (3.11 g, 10.5 mmol) and DIPEA (2.58 g, 20 mmol) were placed in a 30 mL sealed tube, heated to 125°C and stirred for 18 hours. After cooling to room temperature, ethanol (80 mL) was added, and the resulting mixture was stirred, during which water (120 mL) was gradually added dropwise. The resulting mixture was stirred for 30 minutes, filtered to collect the precipitate to produce the target product **6a** (3.97 g, 94%).

MS m/z (ESI): 423 [M+1]

$^1$H NMR (400 MHz, $CDCl_3$) δ 7.62 (d, *J*=8.1 Hz, 2H), 7.40 (d, *J*=8.3 Hz, 2H), 7.32 (d, *J*=8.0 Hz, 2H), 7.11 (d, *J*=8.3 Hz, 2H), 3.32 (q, *J*=6.6 Hz, 1H), 2.97 (s, 4H), 2.61 - 2.49 (m, 2H), 2.49 - 2.36 (m, 2H), 2.44 (s, 3H), 1.27 (d, *J*=6.7 Hz, 3H).

Step 2

*(R)*-1-(1-(4-bromophenyl)ethyl)piperazine (**6b**)

**6a** (1 g, 2.36 mmol) was dissolved in trifluoroacetic acid (2.69 g, 23.6 mmol). Then a concentrated sulfuric acid (1.62 g, 16.5 mmol) was added. The mixture was heated to 75°C and stirred for 16 hours. After cooling to room temperature, a saturated sodium bicarbonate solution (100 mL) was slowly added, and then the mixture was extracted with ethyl acetate (3×100 mL). The combined organic phases were dried over anhydrous sodium sulfate. After filtering, the filtrate was concentrated to dryness under a reduced pressure to produce the target product **6b** (860 mg, crude product). The product was not further purified and directly used in the next step reaction.

MS m/z (ESI): 269 [M+1]

Step 3

*(R)*-4-(4-(1-(4-(bromophenyl)ethyl)piperazin-1-yl)-3-fluorobenzonitrile (**6c**)

**6b** (860 mg, crude product, about 2.36 mmol) and 3,4-difluorobenzonitrile (328 mg, 2.36 mmol) were dissolved in DMF (15 mL). Then potassium carbonate (978 mg, 7.08 mmol) was added. The mixture was heated to 110°C and stirred for 16 hours. After cooling to room temperature, water was added (150 mL) and stirred for 1 hour. The mixture was filtered to collect the precipitate to produce the target product **6c** (506 mg, 55%).
MS m/z (ESI): 388 [M+1]
$^1$H NMR (400 MHz, $CDCl_3$) $\delta$ 7.45 (d, *J*=6.5 Hz, 2H), 7.35 (d, *J*=8.0 Hz, 1H), 7.29-7.15 (m, 3H), 6.88 (t, *J*=8.6 Hz, 1H), 3.39 (d, *J*=5.4 Hz, 1H), 3.19 (s, 4H), 2.63 (s, 2H), 2.54 (s, 2H), 1.36 (d, *J*=6.0 Hz, 3H).

Step 4

Methyl *(R)*-4-(1-(4-(4-cyano-2-fluorophenyl)piperazin-1-yl)ethyl)benzoate (**6d**)

**6c** (506 mg, 1.3 mmol) and methanol (40 mL) were mixed. Then triethylamine (659 mg, 6.5 mmol) was added. The mixture was cooled to -40°C, and bubbled with carbon monoxide for 20 minutes. Then $PdCl_2$(dppf) (190 mg, 0.26 mmol) was added. The mixture was heated to 100°C, stirred for 18 hours, cooled to room temperature, and filtered through Celite. The filtrate was concentrated to dryness under a reduced pressure. The residue was purified by a silica gel column chromatography (petroleum ether/ethyl acetate=100/0 to 1/1) to produce the target product **6d** (360 mg, 75%).
MS m/z (ESI): 368 [M+1]
$^1$H NMR (400 MHz, $CDCl_3$) $\delta$ 8.01 (d, *J*=7.3 Hz, 2H), 7.42 (d, *J*=7.1 Hz, 2H), 7.35 (d, *J*=8.1 Hz, 1H), 7.25 (d, *J*=9.8 Hz, 1H), 6.89 (t, *J*=8.4 Hz, 1H), 3.92 (s, 3H), 3.48 (d, *J*=5.5 Hz, 1H), 3.20 (s, 4H), 2.66 (s, 2H), 2.54 (s, 2H), 1.39 (d, *J*=5.7 Hz, 3H).

Step 5

*(R)*-3-fluoro-4-(4-(1-(4-(hydroxymethyl)phenyl)ethyl)piperazin-1-yl)benzonitrile (**6e**)

**6d** (360 mg, 0.98 mmol) was dissolved in THF (20 mL), and the mixture was cooled to 0°C. Then LAH (45 mg, 1.18 mmol) was added. The mixture was stirred at room temperature for 2 hours, cooled to 0°C, and then quenched with a NaOH solution (1 N, 1 mL). The reaction solution was dried over anhydrous sodium sulfate. After filtering, the filtrate was concentrated to dryness under a reduced pressure. The residue was purified by a silica gel column chromatography (dichloromethane/ethyl acetate=7/3 to 4/1) to produce the target product **6e** (100 mg, 30%).
MS m/z (ESI): 340 [M+1]

Step 6

*(R)*-4-(4-(1-(4-(chloromethyl)phenyl)ethyl)piperazin-1-yl)-3 -fluorobenzonitrile (**6f**)

**6e** (100 mg, 0.29 mmol) was dissolved in dichloromethane (5 mL). Then thionyl chloride (69 mg, 0.58 mmol) was added. The mixture was stirred at room temperature for 16 hours, and concentrated to dryness to produce the target product **6f** (an oily substance, crude product). The product was not further purified and directly used in the next step reaction. MS m/z (ESI): 358 [M+1]

Step 7

Tert-butyl *(S)*-5-amino-4-(4-((4-((R)-1-(4-(4-cyano-2-fluorophenyl)piperazin-1-yl)ethyl)benzyl)ox y)-1-oxoisoindolin-2-yl)-5-oxopentanoate (6g)

**6f** (crude product, about 0.29 mmol) was dissolved in DMF (5 mL). Then **5f** (97 mg, 0.29 mmol) and potassium carbonate (200 mg, 1.45 mmol) were added. The mixture was heated to 50°C, stirred for 20 hours, cooled to room temperature, and diluted with water (80 mL). The resulting mixture was filtered to collect the precipitate to produce the target product **6g** (197 mg, two steps: 104%).
MS m/z (ESI): 656 [M+1]

Step 8

4-(4-((*R*)-1-(4-(((2-((*S*)-2,6-dioxopiperidin-3-yl)-1-oxoisoindolin-4-yl)oxy)methyl)phen yl)ethyl)piperazin-1-yl)-3-fluorobenzonitrile (**6**)

**6g** (197 mg, 0.29 mmol) was dissolved in acetonitrile (10 mL). Then TsOH·$H_2O$ (110 mg, 0.58 mmol) was added. The mixture was heated to 90°C, stirred for 3 hours, cooled to room temperature, and diluted with a saturated sodium bicarbonate solution (30 mL). Then the resulting mixture was extracted with ethyl acetate (3×30 mL). The combined organic phases were dried over anhydrous sodium sulfate. After filtering, the filtrate was concentrated to dryness under a reduced pressure. The residue was purified with a reversed-phase preparative high performance liquid chromatography to produce the target product (21 mg, solid, 12%).

MS m/z (ESI): 582 [M+1]

$^1$H NMR (400 MHz, $CD_3OD$) δ 7.50 - 7.34 (m, 8H), 7.28 (d, *J*=8.0 Hz, 1H), 7.05 (t, *J*=8.6 Hz, 1H), 5.21 (s, 2H), 5.12 (dd, *J*=13.4, 5.2 Hz, 1H), 4.43 (q, *J*=17.4 Hz, 2H), 3.47 (q, *J*=6.7 Hz, 1H), 3.25 - 3.15 (m, 4H), 2.94 - 2.82 (m, 1H), 2.75 (ddd, *J*=17.6, 4.6, 2.4 Hz, 1H), 2.71 - 2.63 (m, 2H), 2.58 - 2.41 (m, 3H), 2.15 (tdd, *J*=7.6, 5.8, 3.1 Hz, 1H), 1.41 (d, *J*=6.7 Hz, 3H).

Compounds 7, 8 and 9 were prepared according to the experimental procedures from step 1 to step 8 for compound 6, but in step 1, different compounds were used to replace *(R)*-1-(4-bromophenyl)ethylamine 3a.

| Compound No. | Compound structure | Compound in replacement of 3**a** | MS m/z (ESI) |
|---|---|---|---|
| **7** | 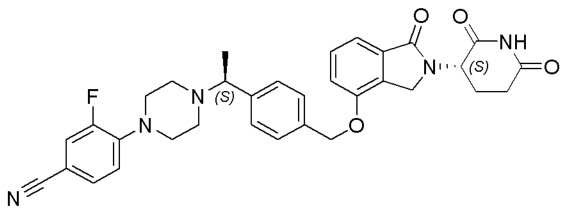 | 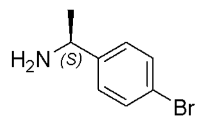 | 582 |
| **8** | 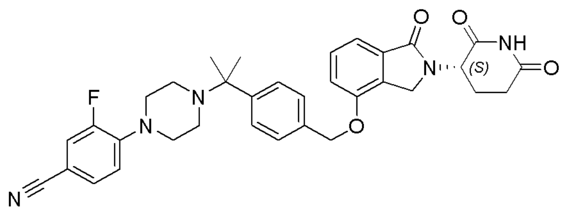 | 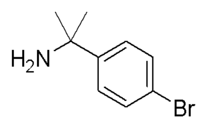 | 596 |
| **9** | 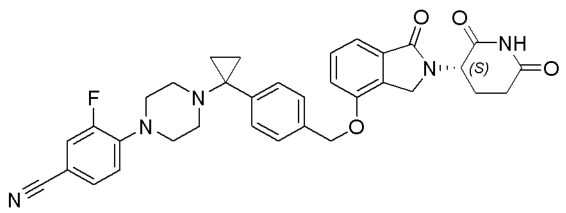 | 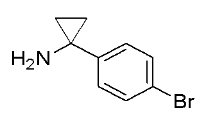 | 594 |

The NMR data of Compounds 7, 8 and 9 were as follows:

| Compound | $^1$H NMR |
|---|---|
| 4-(4-((*S*)-1-(4-(((2-((*S*)-2 ,6-dioxopiperidin-3-yl)-1 -oxoisoindolin-4-yl)oxy) methyl)phenyl)ethyl)pip erazin-1-yl)-3-fluoroben zonitrile (**7**) | $^1$H NMR (400 MHz, $CD_3OD$) δ 7.52 - 7.33 (m, 8H), 7.29 (d, *J*=7.7 Hz, 1H), 7.06 (t, *J*=8.6 Hz, 1H), 5.21 (s, 2H), 5.13 (dd, *J*=13.3, 5.2 Hz, 1H), 4.44 (q, *J*=17.4 Hz, 2H), 3.47 (q, *J*=6.7 Hz, 1H), 3.25 - 3.17 (m, 4H), 2.93 - 2.84 (m, 1H), 2.76 (ddd, *J*=17.6, 4.6, 2.4 Hz, 1H), 2.71 - 2.63 (m, 2H), 2.58 - 2.42 (m, 3H), 2.15 (dtd, *J*=12.8, 5.2, 2.3 Hz, 1H), 1.41 (d, *J*=6.7 Hz, 3H). |

(continued)

| Compound | $^1$H NMR |
|---|---|
| *(S)*-4-(4-(2-(4-(((2-(2,6-d ioxopiperidin-3-yl)-1-ox oisoindolin-4-yl)oxy)met hyl)phenyl)prop-2-yl)pip erazin-1-yl)-3-fluoroben zonitrile (**8**) | $^1$H NMR (400 MHz, $CD_3OD$) δ 7.61 (d, *J*=8.1 Hz, 2H), 7.52 - 7.36 (m, 6H), 7.29 (d, *J*=8.0 Hz, 1H), 7.06 (t, *J*=8.6 Hz, 1H), 5.21 (s, 2H), 5.13 (dd, *J*=13.3, 5.0 Hz, 1H), 4.44 (q, *J*=17.4 Hz, 2H), 3.19 (t, *J*=10.0 Hz, 4H), 3.03 - 2.84 (m, 1H), 2.84 - 2.72 (m, 1H), 2.72 - 2.56 (m, 4H), 2.49 (ddd, *J*=26.8, 13.3, 4.9 Hz, 1H), 2.17 (dd, *J*=15.7, 9.9 Hz, 1H), 1.56 - 1.37 (m, 6H). |
| *(S)*-4-(4-(1-(4-(((2-(2,6-d ioxopiperidin-3-yl)-1-ox oisoindolin-4-yl)oxy)met hyl)phenyl)cyclopropyl) piperazin-1-yl)-3-fluorob enzonitrile (**9**) | $^1$H NMR (400 MHz, $CD_3OD$) δ 7.79 - 7.19 (m, 9H), 7.00 (t, *J*=8.6 Hz, 1H), 5.22 (s, 2H), 5.11 (dd, *J*=13.3, 5.1 Hz, 1H), 4.43 (q, *J*=17.4 Hz, 2H), 3.11 (d, *J*=23.5 Hz, 4H), 2.94 - 2.82 (m, 1H), 2.81 - 2.71 (m, 1H), 2.64 (d, *J*=21.8 Hz, 4H), 2.46 (ddd, *J*=26.3, 13.2, 5.0 Hz, 1H), 2.19 - 2.07 (m, 1H), 1.03 - 0.94 (m, 2H), 0.89 - 0.88 (s, 2H). |

**Example 6**

**2-(4-(((2-(*(S)*-2,6-dioxopiperidin-3-yl)-1-oxoisoindolin-4-yl)oxy)methyl)phenyl)-2-m orpholinoacetonitrile (Compound 10)**

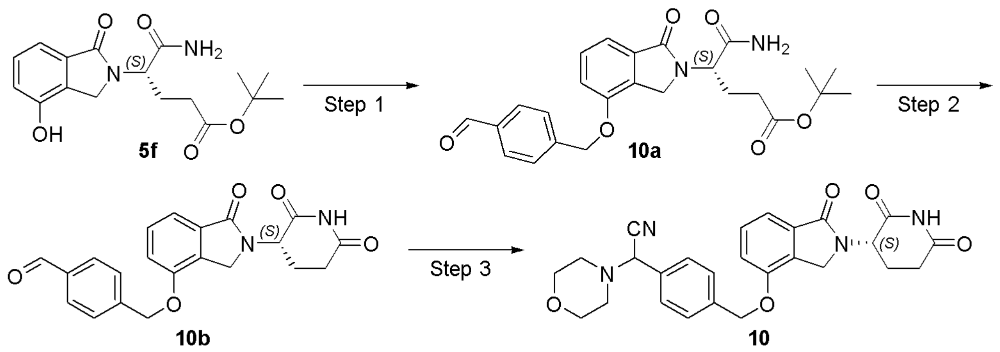

5f, 10a, 10b, 10

Step 1

tert-butyl *(S)*-5-amino-4-(4-((4-formylbenzyl)oxy)-1-oxoisoindolin-2-yl)-5-oxopentanoate (**10a**)

**5f** (167 mg, 0.5 mmol) and 4-(chloromethyl)benzaldehyde (77 mg, 0.5 mmol) was dissolved in DMF (3 mL). Then potassium carbonate (207 mg, 1.5 mmol) was added. The mixture was heated to 50°C, stirred for 18 hours, cooled to room temperature and filtered. The filtrate was concentrated to dryness under a reduced pressure. The residue was purified by a silica gel column chromatography (dichloromethane/methanol=100/0 to 9/1) to produce the target product **10a** (164 mg, 72%).

MS m/z (ESI): 453 [M+1]

Step 2

*(S)*-4-(((2-(2,6-dioxopiperidin-3-yl)-1-oxoisoindolin-4-yl)oxy)methyl)benzaldehyde (**10b**)

**10a** (164 mg, 0.36 mmol) was dissolved in acetonitrile (20 mL). Then $TsOH\cdot H_2O$ (138 mg, 0.72 mmol) was added. The mixture was heated to 90°C, stirred for 18 hours, and cooled to room temperature. The solvent was removed under a reduced pressure. The residue was diluted with a saturated sodium bicarbonate solution (20 mL), and then extracted with ethyl acetate (3×20 mL). The combined organic phases were dried over anhydrous sodium sulfate. After filtering, the filtrate was concentrated to dryness under a reduced pressure. The residue was purified by a silica gel

column chromatography (dichloromethane/methanol=100/0 to 47/3) to produce the target product **10b** (48 mg, 35%).
MS m/z (ESI): 379 [M+1]

Step 3

2-(4-(((2-(*(S)*-2,6-dioxopiperidin-3-yl)-1-oxoisoindolin-4-yl)oxy)methyl)phenyl)-2-mor pholinoacetonitrile (**10**)

**10b** (48 mg, 0.13 mmol) and dichloromethane (10 mL) were mixed. Then morpholine (23 mg, 0.26 mmol), trimethylsilyl cyanide (26 mg, 0.26 mmol) and ytterbium(III) trifluoromethanesulfonate (8 mg, 0.013 mmol) were added. The mixture was stirred at room temperature for 61 hours, and then the solvent was removed under a reduced pressure. The residue was purified by a silica gel column chromatography (dichloromethane/methanol=100/0 to 91/9) and a thin layer silica gel chromatography (dichloromethane/methanol=8/1) to produce the target product **10** (44.6 mg, solid, 72%).
MS m/z (ESI): 475 [M+1]
$^1$H NMR (400 MHz, DMSO-$d_6$) δ 10.97 (s, 1H), 7.58 (d, *J*=8.2 Hz, 2H), 7.52 - 7.46 (m, 3H), 7.33 (dd, *J*=7.7, 3.1 Hz, 2H), 5.40 (s, 1H), 5.28 (s, 2H), 5.12 (dd, *J*=13.3, 5.1 Hz, 1H), 4.43 (d, *J*=17.5 Hz, 1H), 4.27 (d, *J*=17.5 Hz, 1H), 3.66 - 3.52 (m, 4H), 2.96 - 2.85 (m, 1H), 2.58 (d, *J*=19.0 Hz, 2H), 2.48 - 2.34 (m, 4H), 1.99 (dd, *J*=9.0, 3.7 Hz, 1H).

**Example 7**

***(S)*-3-(4-((4-(*(R)*-1-(4-(cyclopropanecarbonyl)piperazin-1-yl)ethyl)benzyl)oxy)-1-ox oisoindolin-2-yl)piperidine-2,6-dione (Compound 11)**

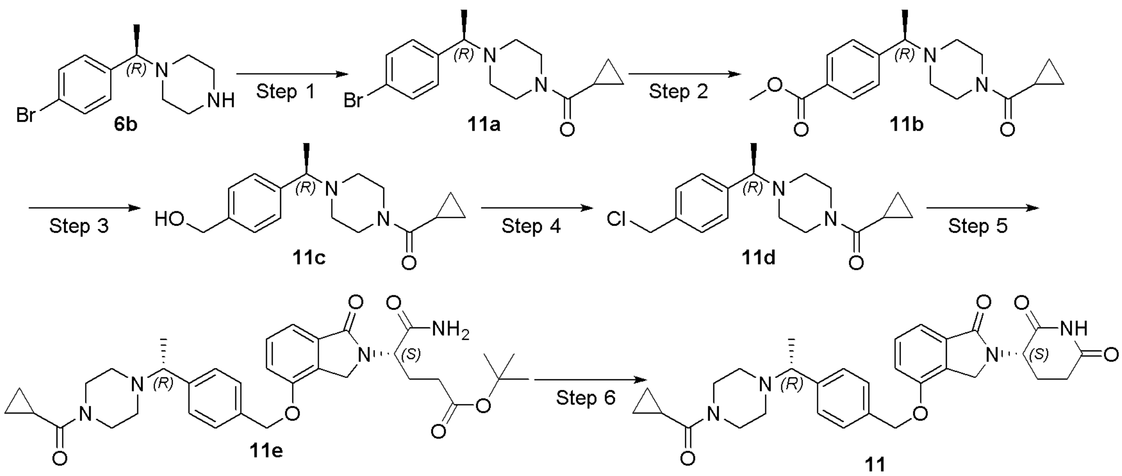


Step 1

*(R)*-(4-(1-(4-bromophenyl)ethyl)piperazin-1-yl)(cyclopropyl)methanone (**11a**)

**6b** (200 mg, 0.75 mmol), cyclopropanecarbonyl chloride (93 mg, 0.89 mmol) and dichloromethane (5 mL) were mixed. Then triethylamine (225 mg, 2.23 mmol) was added. The reaction mixture was stirred at room temperature overnight, and then concentrated to dryness under a reduced pressure. The residue was purified by a silica gel column chromatography (petroleum ether/ethyl acetate=19/1 to 4/1) to produce the target product **11a** (200 mg, 80%).
MS m/z (ESI): 337 [M+1]

Step 2

Methyl *(R)*-4-(1-(4-(cyclopropanecarbonyl)piperazin-1-yl)ethyl)benzoate (**11b**)

**11a** (200 mg, 0.59 mmol), triethylamine (299 mg, 2.97 mmol) and methanol (20 mL) were mixed. The mixture was cooled to -40°C, and then bubbled with carbon monoxide for 30 minutes. Then $PdCl_2$(dppf) (43 mg, 0.06 mmol) was added. The mixture was heated to 100°C in a sealed tube, stirred for 18 hours, and cooled to room temperature. The solvent was removed under a reduced pressure. The residue was purified by a silica gel column chromatography

(petroleum ether/ethyl acetate=9/1 to 3/2) to produce the target product **11b** (120 mg, 75%).

MS m/z (ESI): 317 [M+1]

Step 3

*(R)*-cyclopropyl(4-(1-(4-(hydroxymethyl)phenyl)ethyl)piperazin-1-yl)methanone (**11c**)

To a solution of **11b** (120 mg, 0.38 mmol) in THF (5 mL) was added LAH (22 mg, 0.57 mmol) at 0°C. The mixture was stirred for 1 hour, and then sodium sulfate decahydrate (100 mg) was added. After filtering, the filtrate was concentrated to dryness under a reduced pressure. The residue was purified by a silica gel column chromatography (petroleum ether/ethyl acetate=1/1 to 0/100) to produce the target product **11c** (100 mg, 59%).

MS m/z (ESI): 289 [M+1]

Step 4

*(R)*-(4-(1-(4-(chloromethyl)phenyl)ethyl)piperazin-1-yl)(cyclopropyl)methanone (**11d**)

To a solution of **11c** (100 mg, 0.35 mmol) in dichloromethane (5 mL) was added thionyl chloride (0.5 mL). The reaction mixture was stirred at room temperature overnight, and then the solvent was removed under a reduced pressure to produce the target product **11d** (100 mg, crude product). The product was not further purified and directly used in the next step reaction.

MS m/z (ESI): 307 [M+1]

Step 5

Tert-butyl (S)-5-amino-4-(4-((4-((R)-1-(4-(cyclopropanecarbonyl)piperazin-1-yl)ethyl)benzyl)oxy) -1-oxoisoindolin-2-yl)-5-oxopentanoate (**11e**)

**11d** (100 mg, crude product), **5f** (101 mg, 0.30 mmol), potassium carbonate (83 mg, 0.60 mmol) and DMF (5 mL) were mixed. The mixture was heated to 50°C, stirred overnight, cooled to room temperature, and diluted with water (10 mL). The resulting mixture was extracted with ethyl acetate (3×20 mL). The combined organic phases were dried over anhydrous sodium sulfate, and filtered. The resulting filtrate was concentrated to dryness under a reduced pressure. The residue was purified by a silica gel column chromatography (dichloromethane/methanol=100/0 to 19/1) to produce the target product **11e** (100 mg, 53%).

MS m/z (ESI): 605 [M+1]

Step 6

*(S)*-3-(4-((4-(*(R)*-1-(4-(cyclopropanecarbonyl)piperazin-1-yl)ethyl)benzyl)oxy)-1-oxoisoindolin-2-yl)piperidine-2,6-dione (**11**)

**11e** (100 mg, 0.17 mmol), $TsOH \cdot H_2O$ (66 mg, 0.35 mmol) and acetonitrile (2 mL) were mixed. The mixture was heated to 90°C, stirred overnight, and cooled to room temperature. The solvent was removed under a reduced pressure. The residue was purified with a reversed-phase preparative high performance liquid chromatography to produce the target product **11** (10 mg, solid, 22%).

MS m/z (ESI): 531 [M+1]

$^1H$ NMR (400 MHz, $CD_3OD$) $\delta$ 7.54 - 7.32 (m, 6H), 7.29 (d, *J*=7.9 Hz, 1H), 5.22 (s, 2H), 5.13 (dd, *J*=13.4, 5.2 Hz, 1H), 4.45 (q, *J*=17.3 Hz, 2H), 3.74 (s, 1H), 3.57 (s, 1H), 3.50 - 3.41 (m, 1H), 3.01 - 2.83 (m, 2H), 2.83 - 2.71 (m, 1H), 2.62 - 2.42 (m, 4H), 2.37 (s, 1H), 2.22 - 2.10 (m, 1H), 1.90 (ddd, *J*=12.8, 7.9, 4.7 Hz, 1H), 1.40 (d, *J*=6.7 Hz, 3H), 1.36 - 1.26 (m, 1H), 0.87 - 0.73 (m, 4H).

**Example 8**

***(S)*-3-(4-((4-(*(R)*-1-(4-cyclopropylpiperazin-1-yl)ethyl)benzyl)oxy)-1-oxoisoindolin-2-yl)piperidine-2,6-dione (Compound 12)**

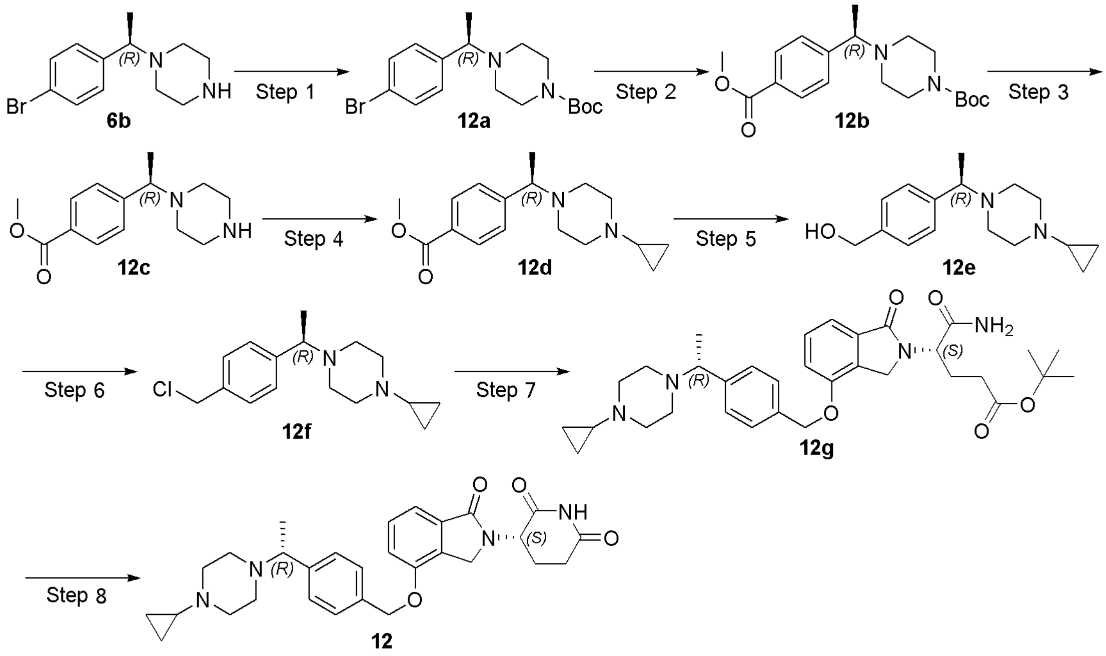


Step 1

Tert-butyl *(R)*-4-(1-(4-bromophenyl)ethyl)piperazine-1-carboxylate (**12a**)

**6b** (*p*-toluenesulfonate, 4 g, 9 mmol) and THF (80 mL) were mixed. Then triethylamine (3.7 g, 36 mmol) was added. The mixture was cooled to 0°C, and di-tert-butyl dicarbonate (2.2 g, 9.9 mmol) was added dropwise. The mixture was stirred at room temperature for 17 hours, and water was added (160 mL). Then the resulting mixture was extracted with ethyl acetate (2×100 mL). The combined organic phases were dried over anhydrous sodium sulfate. After filtering, the filtrate was concentrated to dryness. The residue was purified by a silica gel column chromatography (petroleum ether/ethyl acetate=100/0 to 1/1) to produce the target product **12a** (3.07 g, 92%).

MS m/z (ESI): 369 [M+1]

Step 2

tert-butyl *(R)*-4-(1-(4-(methoxycarbonyl)phenyl)ethyl)piperazin-1-carboxylate (**12b**)

**12a** (3.07 g, 8.3 mmol) was dissolved in methanol (120 mL) in a sealed tube, and triethylamine (4.21 g, 41.6 mmol) was added. The mixture was cooled to -40°C, and bubbled with carbon monoxide for 30 minutes. Then $PdCl_2$(dppf) (1.21 g, 1.66 mmol) was added. The mixture was heated to 100°C, stirred for 18 hours, cooled to room temperature, and filtered through a Celite pad. The filtrate was concentrated to dryness under a reduced pressure. The residue was purified by a silica gel column chromatography (petroleum ether/ethyl acetate=100/0 to 1/1) to produce the target product **12b** (2.7 g, 93%).

MS m/z (ESI): 349 [M+1]

Step 3

Methyl *(R)*-4-(1-(piperazin-1-yl)ethyl)benzoate (**12c**)

To a solution of **12b** (350 mg, 1.01 mmol) in dichloromethane (4 mL) was added trifluoroacetic acid (1 mL). The mixture was stirred at room temperature for 4 hours, and the solvent was removed under a reduced pressure to produce the target product **12c** (350 mg, crude product). The product was not further purified and directly used in the next step reaction.

MS m/z (ESI): 249 [M+1]

Step 4

Methyl *(R)*-4-(1-(4-(cyclopropylpiperazin-1-yl)ethyl)benzoate (**12d**)

To a mixture of **12c** (140 mg, 0.56 mmol), (1-ethoxycyclopropyloxy)trimethylsilane (295 mg, 1.69 mmol), acetic acid (100 mg, 1.69 mmol) and methanol (5 mL) were added sodium cyanoborohydride (106 mg, 1.69 mmol). The reaction mixture was stirred at room temperature overnight, and the solvent was removed under a reduced pressure. The residue was purified by a silica gel column chromatography (petroleum ether/ethyl acetate=9/1 to7/3) to produce the target product **12d** (100 mg, 62%).

MS m/z (ESI): 289 [M+1]

Step 5

*(R)*-(4-(1-(4-(cyclopropylpiperazin-1-yl)ethyl)phenyl)methanol (**12e**)

To a solution of **12d** (100 mg, 0.35 mmol) in THF (3 mL) was added LAH (20 mg, 0.52 mmol) at 0°C. The mixture was stirred for 1 hour, and sodium sulfate decahydrate (100 mg) was added. After filtering, the filtrate was concentrated to dryness under a reduced pressure. The residue was purified by a silica gel column chromatography (dichloromethane/methanol=99/1 to 19/1) to produce the target product **12e** (75 mg, 83%).

MS m/z (ESI): 261 [M+1]

Step 6

*(R)*-1-(1-(4-(chloromethyl)phenyl)ethyl)-4-cyclopropylpiperazine (**12f**)

To a solution of **12e** (80 mg, 0.31 mmol) in dichloromethane (5 mL) was added thionyl chloride (1 mL). The mixture was stirred at room temperature overnight, and the solvent was removed under a reduced pressure to produce the target product **12f** (80 mg, crude product). The product was not further purified and directly used in the next step reaction. MS m/z (ESI): 279 [M+1]

Step 7

Tert-butyl *(S)*-5-amino-4-(4-((4-(*(R)*-1-(4-cyclopropylpiperazin-1-yl)ethyl)benzyl)oxy)-1-oxoisoin dolin-2-yl)-5-oxopentanoate (**12g**)

**12f** (80 mg, crude product), **5f** (101 mg, 0.30 mmol), potassium carbonate (83 mg, 0.60 mmol) and DMF (5 mL) were mixed. The mixture was heated to 50°C, stirred overnight, cooled to room temperature, and diluted with water (10 mL). Then the resulting mixture was extracted with ethyl acetate (3×20 mL). The organic phases were combined, and dried over anhydrous sodium sulfate. After filtering, the filtrate was concentrated to dryness under a reduced pressure to produce the target product **12g** (120 mg, crude product). The product was not further purified and directly used in the next step reaction. MS m/z (ESI): 577 [M+1]

Step 8

*(S)*-3-(4-((4-(*(R)*-1-(4-cyclopropylpiperazin-1-yl)ethyl)benzyl)oxy)-1-oxoisoindolin-2-y l)piperidine-2,6-dione formate (**12**)

**12g** (120 mg, crude product), $TsOH \cdot H_2O$ (95 mg, 0.50 mmol) and acetonitrile (2 mL) were mixed. The mixture was heated to 90°C, stirred overnight, and cooled to room temperature. The solvent was removed under a reduced pressure. The residue was purified with a reversed-phase preparative high performance liquid chromatography to produce the target product **12** (10.0 mg, solid, 6.5%).

MS m/z (ESI): 503 [M+1]

$^1$H NMR (400 MHz, $CD_3OD$) $\delta$ 8.45 (s, 1H), 7.48 (dd, *J*=12.7, 7.9 Hz, 3H), 7.40 (d, *J*=7.8 Hz, 3H), 7.28 (d, *J*=8.0 Hz, 1H), 5.24 (s, 2H), 5.14 (dd, *J*=13.3, 5.1 Hz, 1H), 4.45 (q, *J*=17.3 Hz, 2H), 3.79 (dd, *J*=13.4, 6.6 Hz, 1H), 2.98 - 2.71 (m, 8H), 2.67 (d, *J*=15.8 Hz, 2H), 2.56 - 2.45 (m, 1H), 2.16 (ddd, *J*=10.3, 5.2, 2.9 Hz, 1H), 1.85 - 1.76 (m, 1H), 1.51 (d, *J*=6.8 Hz, 3H), 0.50 (dd, *J*=12.1, 5.7 Hz, 2H), 0.48 - 0.38 (m, 2H).

**Example 9**

***(S)*-3-(4-((4-(*(R)*-1-(4-(methylsulfonyl)piperazin-1-yl)ethyl)benzyl)oxy)-1-oxoisoind olin-2-yl)piperidine-2,6-dione (Compound 13)**

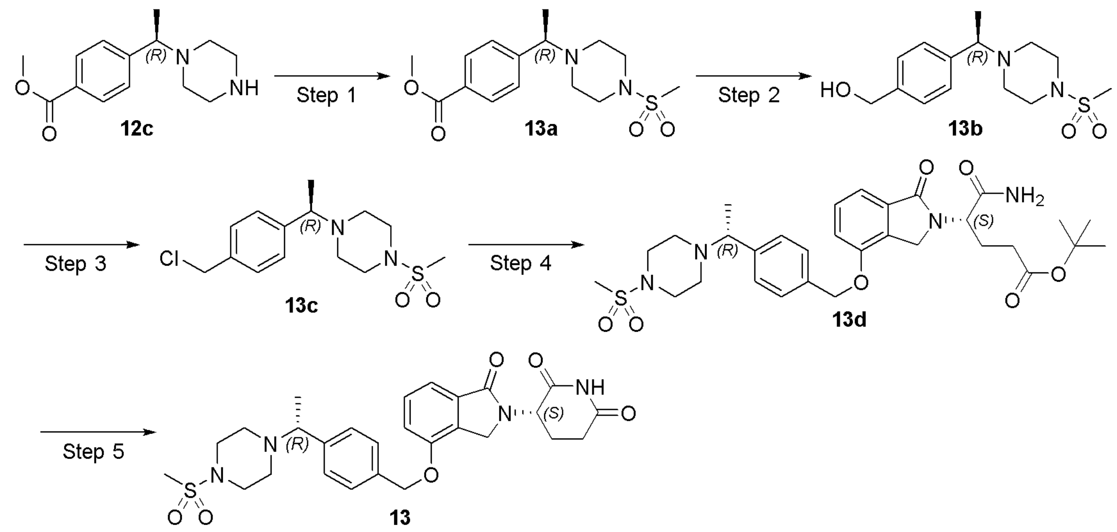


Step 1

Methyl *(R)*-4-(1-(4-(methylsulfonyl)piperazin-1-yl)ethyl)benzoate (**13a**)

**12c** (140 mg, 0.56 mmol), methanesulfonyl chloride (97 mg, 0.85 mmol), triethylamine (90 mg, 0.90 mmol) and dichloromethane (5 mL) were mixed. The mixture was stirred overnight, and the solvent was removed under a reduced pressure. The residue was purified by a silica gel column chromatography (petroleum ether/ethyl acetate=9/1 to 1/1) to produce the target product **13a** (100 mg, 54%).

MS m/z (ESI): 327 [M+1]

Step 2

*(R)*-(4-(1-(4-(methylsulfonyl)piperazin-1-yl)ethyl)phenyl)methanol (**13b**)

To a solution of **13a** (100 mg, 0.31 mmol) in THF (3 mL) was added LAH (18 mg, 0.46 mmol) at 0°C. The mixture was stirred for 1 hour, and then sodium sulfate decahydrate (100 mg) was added. After filtering, the filtrate was concentrated to dryness under a reduced pressure. The residue was purified by a silica gel column chromatography (dichloromethane/methanol=1% to 5%) to produce the target product **13b** (80 mg, 88%). MS m/z (ESI): 299 [M+1]

Step 3

*(R)*-1-(1-(4-(chloromethyl)phenyl)ethyl)-4-(methylsulfonyl)piperazine (**13c**)

To a solution of **13b** (40 mg, 0.13 mmol) in dichloromethane (5 mL) was added thionyl chloride (1 mL). The mixture was stirred at room temperature overnight, and the solvent was removed under a reduced pressure to produce the target product **13c** (40 mg, crude product). The product was not further purified and directly used in the next step reaction. MS m/z (ESI): 317 [M+1]

Step 4

Tert-butyl *(S)*-5-amino-4-(4-((4-(*(R)*-1-(4-(methylsulfonyl)piperazin-1-yl)ethyl)benzyl)oxy)-1-oxoi soindolin-2-yl)-5-oxopentanoate (**13d**)

**13c** (40 mg, crude product), **5f** (101 mg, 0.30 mmol), potassium carbonate (83 mg, 0.60 mmol) and DMF (5 mL) were mixed. The mixture was heated to 50°C, stirred overnight, cooled to room temperature, and diluted with water (20 mL). Then the mixture was extracted with ethyl acetate (3×20 mL). The combined organic phases were dried over anhydrous sodium sulfate, and filtered. The resulting filtrate was concentrated to dryness under a reduced pressure to produce the target product **13d** (60 mg, crude product). The product was not further purified and directly used in the next step reaction.

MS m/z (ESI): 615 [M+1]

Step 5

*(S)*-3-(4-((4-(*(R)*-1-(4-(methylsulfonyl)piperazin-1-yl)ethyl)benzyl)oxy)-1-oxoisoindoli n-2-yl)piperidine-2,6-dione (**13**)

**13d** (60 mg, crude product), TsOH·$H_2O$ (95 mg, 0.50 mmol) and acetonitrile (2 mL) were mixed. The mixture was heated to 90°C, stirred overnight, cooled to room temperature, and concentrated to dryness under a reduced pressure. The residue was purified with a reversed-phase preparative high performance liquid chromatography to produce the target product **13** (9.2 mg, solid, 13%).

MS m/z (ESI): 541 [M+1]

$^1$H NMR (400 MHz, $CD_3OD$) δ 7.52 - 7.33 (m, 6H), 7.29 (d, *J*=8.0 Hz, 1H), 5.21 (s, 2H), 5.13 (dd, *J*=13.4, 5.2 Hz, 1H), 4.44 (q, *J*=17.4 Hz, 2H), 3.50 (q, *J*=6.7 Hz, 1H), 3.19 (t, *J*=4.9 Hz, 4H), 2.97 - 2.84 (m, 1H), 2.81 (s, 3H), 2.76 (ddd, *J*=17.6, 4.5, 2.3 Hz, 1H), 2.61 (dt, J 10.0, 5.0 Hz, 2H), 2.48 (dd, *J*=10.6, 4.9 Hz, 3H), 2.25 - 2.10 (m, 1H), 1.39 (d, *J*=6.7 Hz, 3H).

**Example 10**

***(S)*-3-(4-((4-(*(R)*-1-(4-methylpiperazin-1-yl)ethyl)benzyl)oxy)-1-oxoisoindolin-2-yl)p iperidine-2,6-dione (Compound 14)**

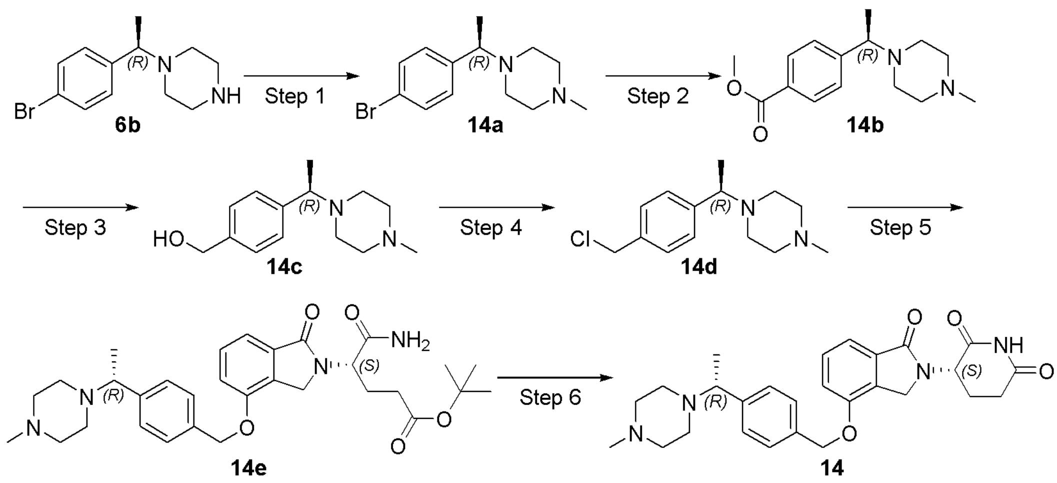


Step 1

*(R)*-1-(1-(4-(bromophenyl)ethyl)-4-methylpiperazine (**14a**)

**6b** (200 mg, 0.75 mmol), an aqueous formaldehyde solution (40%, 0.3 mL), acetic acid (122 mg, 2.0 mmol) and methanol (10 mL) were mixed. Then sodium cyanoborohydride (127 mg, 2.0 mmol) was added. The mixture was stirred at room temperature overnight, and the solvent was removed under a reduced pressure. The residue was purified

by a silica gel column chromatography (petroleum ether/ethyl acetate=3/2 to 1/4) to produce the target product **14a** (200 mg, 95%).

MS m/z (ESI): 283 [M+1]

Step 2 to Step 6

*(S)*-3-(4-((4-(*(R)*-1-(4-methylpiperazin-1-yl)ethyl)benzyl)oxy)-1-oxoisoindolin-2-yl)pip eridine-2,6-dione (**14**)

Compound **14** was prepared according to the experimental procedures from step 2 to step 6 for compound **11,** but in step 2, **14a** was used to replace **11a.**

MS m/z (ESI): 283 [M+1]

$^1$H NMR (400 MHz, $CD_3OD$) δ 7.55 - 7.43 (m, 3H), 7.38 (t, *J*=9.6 Hz, 3H), 7.28 (d, *J*=7.7 Hz, 1H), 5.23 (s, 2H), 5.14 (d, *J*=8.6 Hz, 1H), 4.44 (q, *J*=17.3 Hz, 2H), 3.58 (dd, *J*=12.0, 5.9 Hz, 1H), 3.03 (d, *J*=27.6 Hz, 4H), 2.96 - 2.73 (m, 4H), 2.70 (s, 3H), 2.64 (s, 2H), 2.54 - 2.42 (m, 1H), 2.17 (dt, *J*=8.5, 6.3, 1H), 1.41 (d, *J*=6.3 Hz, 3H).

**Example 11**

***(S)*-3-(1-oxo-4-((4-(*(R)*-1-(4-(pyrimidin-4-yl)piperazin-1-yl)ethyl)benzyl)oxy)isoindo lin-2-yl)piperidine-2,6-dione (Compound 15)**

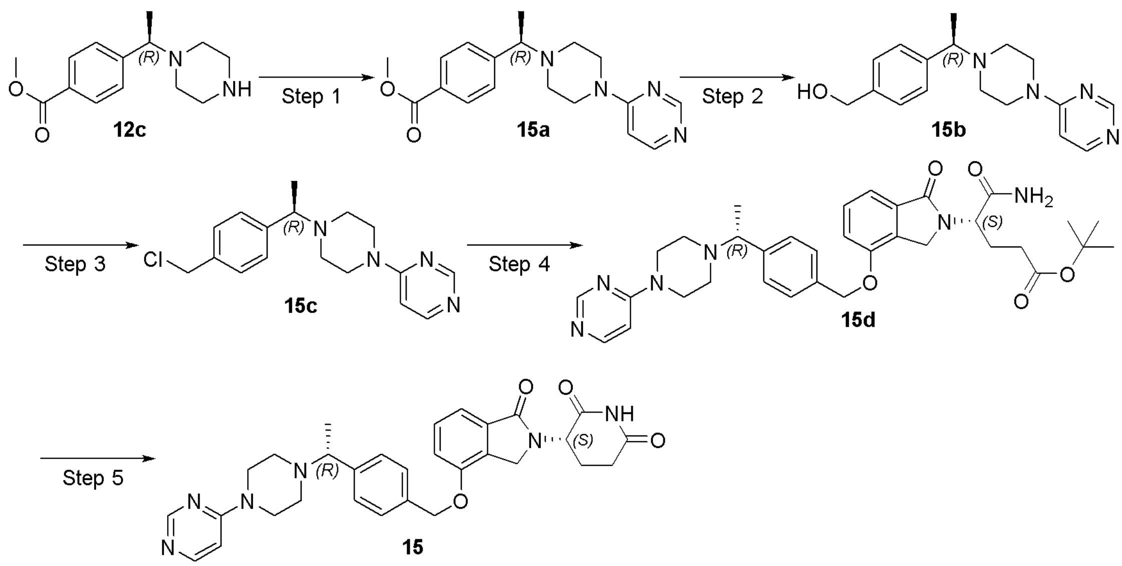


12c, 15a, 15b, 15c, 15d, 15

Step 1

Methyl *(R)*-4-(1-(4-(pyrimidin-4-yl)piperazin-1-yl)ethyl)benzoate (**15a**)

**12c** (200 mg, 0.81 mmol), 4-chloropyrimidine hydrochloride (243 mg, 1.61 mmol), potassium carbonate (334 mg, 2.42 mmol) and DMF (2 mL) were mixed. The mixture was heated to 110°C, stirred overnight, cooled to room temperature, and diluted with water (10 mL). Then the resulting mixture was extracted with ethyl acetate (3×20 mL). The combined organic phases were dried over anhydrous sodium sulfate. After filtering, the filtrate was concentrated to dryness under a reduced pressure. The residue was purified by a silica gel column chromatography (petroleum ether/ethyl acetate=9/1 to 1/1) to produce the target product **15a** (100 mg, 38%).

MS m/z (ESI): 327 [M+1]

Step 2

*(R)*-(4-(1 -(4-(pyrimidin-4-yl)piperazin-1-yl)ethyl)phenyl)methanol (**15b**)

To a solution of **15a** (100 mg, 0.31 mmol) in THF (5 mL) was added LAH (18 mg, 0.46 mmol) at 0°C. The

mixture was stirred for 1 hour, and then sodium sulfate decahydrate (100 mg) was added. After filtering, the filtrate was concentrated to dryness under a reduced pressure. The residue was purified by a silica gel column chromatography (dichloromethane/methanol=99/1 to 19/1) to produce the target product **15b** (40 mg, 44%).

MS m/z (ESI): 299 [M+1]

Step 3

*(R)*-4-(4-(1-(4-(chloromethyl)phenyl)ethyl)piperazin-1-yl)pyrimidine (**15c**)

To a solution of **15b** (40 mg, 0.13 mmol) in dichloromethane (4 mL) was added thionyl chloride (1 mL). The reaction mixture was stirred overnight, and concentrated under a reduced pressure to dryness to produce the target product **15c** (40 mg, crude product). The product was not further purified and directly used in the next step reaction.

MS m/z (ESI): 317 [M+1]

Step 4

Tert-butyl *(S)*-5-amino-5-oxo-4-(1-oxo-4-((4-(*(R)*-1-(4-(pyrimidin-4-yl)piperazin-1-yl)ethyl)benzy l)oxy)isoindolin-2-yl)pentanoate (**15d**)

**15c** (40 mg, crude product), **5f** (67 mg, 0.20 mmol), potassium carbonate (56 mg, 0.40 mmol) and DMF (1 mL) were mixed. The mixture was heated to 50°C, stirred overnight, cooled to room temperature, and diluted with water (10 mL). Then the mixture was extracted with ethyl acetate (3×10 mL). The combined organic phases were dried over anhydrous sodium sulfate, and filtered. The resulting filtrate was concentrated to dryness under a reduced pressure. The residue was purified by a silica gel column chromatography (dichloromethane/methanol=99/1 to 19/1) to produce the target product **15d** (40 mg, two steps: 49%).

MS m/z (ESI): 615 [M+1]

Step 5

*(S)*-3-(1-oxo-4-((4-(*(R)*-1-(4-(pyrimidin-4-yl)piperazin-1-yl)ethyl)benzyl)oxy)isoindolin -2-yl)piperidine-2,6-dione (**15**)

**15d** (40 mg, 0.07 mmol), $TsOH \cdot H_2O$ (37 mg, 0.20 mmol) and acetonitrile (1 mL) were mixed. The mixture was heated to 90°C, stirred overnight, cooled to room temperature, and concentrated to dryness under a reduced pressure. The residue was purified with a reversed-phase preparative high performance liquid chromatography to produce the target product (20.0 mg, solid, 57%).

MS m/z (ESI): 541 [M+1]

$^1H$ NMR (400 MHz, $CD_3OD$) δ 8.52 (s, 1H), 8.14 (d, *J*=6.8 Hz, 1H), 7.70 (d, *J*=8.2 Hz, 1H), 7.50 (t, *J*=8.6 Hz, 2H), 7.47 - 7.37 (m, 2H), 7.29 (d, *J*=7.7 Hz, 1H), 7.23 (d, *J*=7.9 Hz, 1H), 6.88 (d, *J*=6.9 Hz, 1H), 5.25 (s, 2H), 5.14 (dd, *J*=13.3, 5.2 Hz, 1H), 4.45 (q, *J*=17.3 Hz, 2H), 3.85 (s, 4H), 3.00 - 2.66 (m, 6H), 2.60-2.41 (m, 1H), 2.36 (s, 1H), 2.24 - 2.09 (m, 1H), 1.53 (d, *J*=6.7 Hz, 3H).

Compounds **16** and **20** were prepared according to the experimental procedures from step 1 to step 5 for compound 15, but in step 1, different compounds were used to replace 4-chloropyrimidine hydrochloride.

| Compound No. | Compound structure | Compound in replacement of 4-chloropyrimidin e hydrochloride | MS m/z (ESI) |
|---|---|---|---|
| **16** | 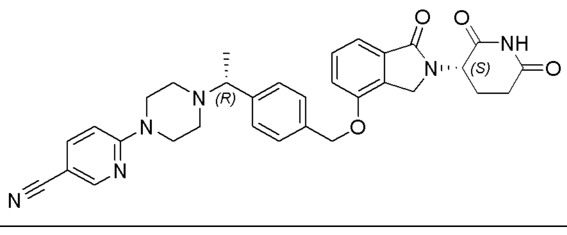 | 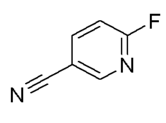 | 565 |

(continued)

| Compound No. | Compound structure | Compound in replacement of 4-chloropyrimidin e hydrochloride | MS m/z (ESI) |
|---|---|---|---|
| **20** | 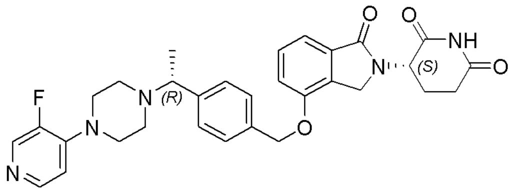 | 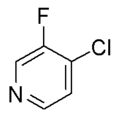 | 558 |

The NMR data of Compounds 16 and 20 were as follows:

| Compound | $^1$H NMR |
|---|---|
| 6-(4-((*R*)-1-(4-(((2-((*S*)-2 ,6-dioxopiperidin-3-yl)-1 -oxoisoindolin-4-yl)oxy) methyl)phenyl) ethyl)pip erazin-1-yl)nicotinonitril e (**16**) | $^1$H NMR (400 MHz, $CD_3OD$) δ 8.36 (s, 1H), 7.69 (d, *J*=9.0 Hz, 1H), 7.52 - 7.34 (m, 6H), 7.29 (d, *J*=8.0 Hz, 1H), 6.80 (d, *J*=9.1 Hz, 1H), 5.22 (s, 2H), 5.13 (dd, *J*=13.4, 5.0 Hz, 1H), 4.44 (q, *J*=17.3 Hz, 2H), 3.68 (t, *J*=4.8 Hz, 4H), 3.56 - 3.46 (m, 1H), 3.03 - 2.71 (m, 3H), 2.65 (dd, *J*=17.8, 11.8 Hz, 2H), 2.57 - 2.44 (m, 2H), 2.23 - 2.10 (m, 1H), 1.43 (d, *J*=6.6 Hz, 3H). |
| *(S)*-3-(4-((4-((R)-1-(4-(3-fluoropyridin-4-yl) pipera zin-1-yl)ethyl)benzyl)ox y)-1-oxoisoindolin-2-yl) piperidine-2,6-dione (**20**) | $^1$H NMR (400 MHz, $CD_3OD$) δ 8.11 (dd, *J*=29.1, 5.2 Hz, 2H), 7.48 (t, *J*=7.5 Hz, 3H), 7.44 - 7.36 (m, 3H), 7.29 (d, *J*=8.0 Hz, 1H), 6.97 (d, *J*=6.4 Hz, 1H), 5.24 (s, 2H), 5.13 (dd, *J*=13.3, 5.0 Hz, 1H), 4.45 (q, *J*=17.3 Hz, 2H), 3.68 (t, *J*=11.7 Hz, 1H), 3.42 (s, 4H), 3.04 - 2.59 (m, 6H), 2.49 (dd, *J*=13.1, 4.2 Hz, 1H), 2.17 (dd, *J*=15.2, 9.8 Hz, 1H), 1.49 (d, *J*=6.8 Hz, 3H). |

**Example 12**

***(S)*-3-(4-((4-(*(R)*-1-(4-(2,4-difluorophenyl)piperazin-1-yl)ethyl)benzyl)oxy)-1-oxoiso indolin-2-yl)piperidine-2,6-dione (Compound 17)**

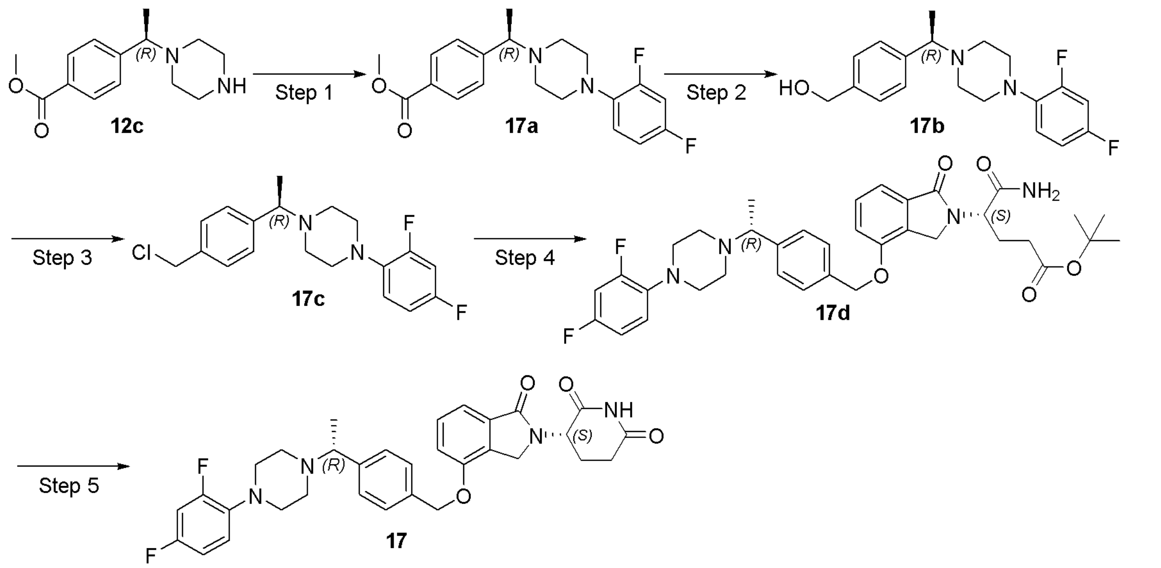

Step 1

Methyl *(R)*-4-(1-(4-(2,4-difluorophenyl)piperazin-1-yl)ethyl)benzoate (**17a**)

**12c** (150 mg, 0.60 mmol), 1-bromo-2,4-difluorobenzene (232 mg, 1.20 mmol), potassium t-butoxide (202 mg, 1.80 mmol), tris(dibenzylideneacetone)dipalladium (35 mg, 0.06 mmol), XPhos (58 mg, 0.12 mmol) and toluene (2 mL) were mixed. Then the mixture was heated under a nitrogen gas atmosphere with a microwave reactor to 130°C, stirred for 1 hour, and cooled to room temperature. The solvent was removed under a reduced pressure. The residue was purified by a silica gel column chromatography (petroleum ether/ethyl acetate = 9/1 to 1/1) to produce the target product **17a** (40 mg, 19%).
MS m/z (ESI): 361 [M+1]

Step 2 to Step 5

*(S)*-3-(4-((4-(*(R)*-1-(4-(2,4-difluorophenyl)piperazin-1-yl)ethyl)benzyl)oxy)-1-oxoisoind olin-2-yl)piperidine-2,6-dione (**17**)

Compound **17** was prepared according to the experimental procedures from step 2 to step 5 for compound 15, but in step 2, **17a** was used to replace **15a.**
MS m/z (ESI): 575 [M+1]
$^1$H NMR (400 MHz, $CD_3OD$) δ 7.54 - 7.44 (m, 3H), 7.43 - 7.35 (m, 3H), 7.30 (d, *J*=8.0 Hz, 1H), 7.07 - 6.98 (m, 1H), 6.93 - 6.82 (m, 2H), 5.23 (s, 2H), 5.13 (dd, *J*=13.4, 5.2 Hz, 1H), 4.45 (q, *J*=17.3 Hz, 2H), 3.57 (dd, *J*=18.3, 11.8 Hz, 1H), 3.04 (t, *J*=4.7 Hz, 3H), 2.95 - 2.84 (m, 1H), 2.82 - 2.70 (m, 3H), 2.66 - 2.58 (m, 2H), 2.57 - 2.42 (m, 2H), 2.21 - 2.12 (m, 1H), 1.45 (d, *J*=6.7 Hz, 3H).

**Example 13**

**(3S)-3-(4-((4-(1-(2-oxa-5-azabicyclo[2.2.1]heptan-5-yl)ethyl)benzyl)oxy)-1-oxoisoin dolin-2-yl)piperidine-2,6-dione (Compounds 18 and 19)**

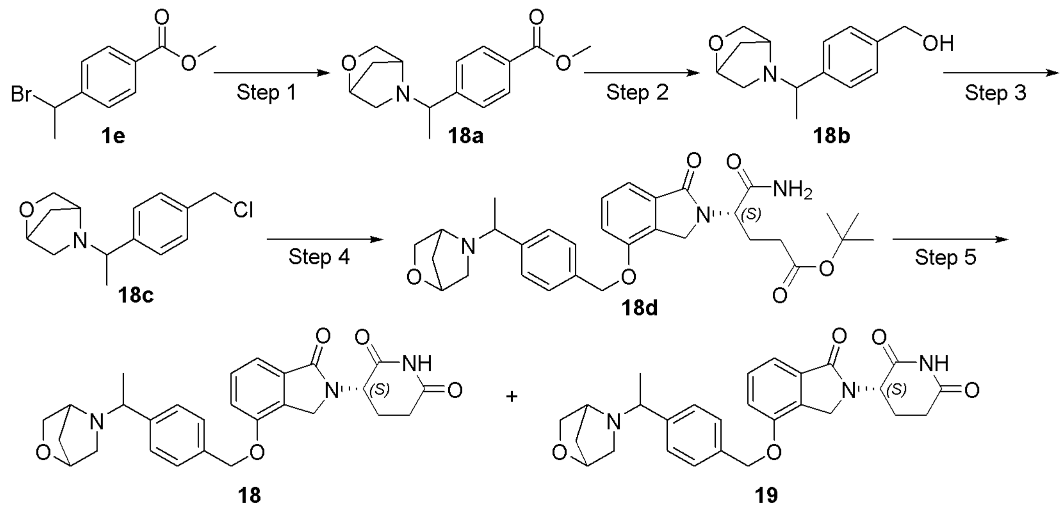


Step 1

Methyl 4-(1-(2-oxa-5-azabicyclo[2.2.1]heptan-5-yl)ethyl)benzoate (**18a**)

Methyl 4-(1-bromoethyl)benzoate **1e** (243 mg, 1 mmol) and 2-oxa-5-azabicyclo[2.2.1]heptane hydrochloride (136 mg, 1 mmol) were dissolved in DMF (4 mL). Then potassium carbonate (553 mg, 4 mmol) was added. The mixture was stirred at room temperature for 19 hours, and diluted with water (50 mL). The resulting mixture was extracted with ethyl acetate (3×30 mL). The combined organic phases were washed with water (3×30mL), and then dried over anhydrous sodium sulfate. After filtering, the filtrate was concentrated to dryness under a reduced pressure. The residue was purified by a silica gel column chromatography (petroleum ether/ethyl acetate=100/0 to 4/1) to produce the target product **18a** (212 mg, 81%).
MS m/z (ESI): 262 [M+1]

Step 2

(4-(1-(2-oxa-5-azabicyclo[2.2.1]heptan-5-yl)ethyl)phenyl)methanol (**18b**)

**18a** (212 mg, 0.81 mmol) was dissolved in THF (5 mL). The mixture was cooled to 0°C. Then LAH (46 mg, 1.22 mmol) was added. The mixture was stirred at 0°C for 30 minutes, then quenched with an aqueous NaOH solution (2.5 N, 1 mL), and dried over anhydrous sodium sulfate. After filtering, the filtrate was concentrated to dryness under a reduced pressure. The residue was purified by a silica gel column chromatography (dichloromethane/methanol=100/0 to 19/1) to produce the target product **18b** (158 mg, 84%).
MS m/z (ESI): 234 [M+1]

Step 3

5-(1-(4-(chloromethyl)phenyl)ethyl)-2-oxa-5-azabicyclo[2.2.1]heptane (**18c**)

To a solution of **18b** (158 mg, 0.68 mmol) in dichloromethane (5 mL) was added thionyl chloride (161 mg, 1.35 mmol). The mixture was stirred at room temperature for 2 hours, and then the solvent was removed under a reduced pressure to produce the target product **18c** (crude product). The product was not further purified and directly used in the next step reaction.
MS m/z (ESI): 252 [M+1]

Step 4

Tert-butyl (4S)-4-(4-((4-(1-(2-oxa-5-azabicyclo[2.2.1]heptan-5-yl)ethyl)benzyl)oxy)-1-oxoisoindol in-2-yl)-5-amino-5-oxopentanoate (**18d**)

**18c** (crude product, about 0.68 mmol), **5f** (226 mg, 0.68 mmol), potassium carbonate (376 mg, 2.72 mmol) and DMF (5 mL) were mixed. The mixture was heated to 50°C, stirred for 48 hours, cooled to room temperature, and diluted with water (80 mL). Then the mixture was filtered to collect the precipitate to produce the target product **18d** (280 mg, two steps: 75%).
MS m/z (ESI): 550 [M+1]

Step 5

(3S)-3-(4-((4-(1-(2-oxa-5-azabicyclo[2.2.1]heptan-5-yl)ethyl)benzyl)oxy)-1-oxoisoindol in-2-yl)piperidine-2,6-dione (**18** and **19**)

To a solution of **18d** (280 mg, 0.51 mmol) in acetonitrile (30 mL) was added TsOH·$H_2O$ (194 mg, 1.02 mmol). The mixture was heated to 90°C, stirred for 18 hours, and cooled to room temperature. The solvent was removed under a reduced pressure. Then a saturated sodium bicarbonate solution (50 mL) was added. The mixture was extracted with ethyl acetate (2×50 mL). The combined organic phases were dried over anhydrous sodium sulfate. After filtering, the filtrate was concentrated to dryness under a reduced pressure. The residue was purified with a reversed-phase preparative high performance liquid chromatography to produce the target product **18** (isomer 1, 45 mg, solid, 19%) and **19** (isomer 2, 19 mg, solid, 8%).
The characterization data of **18** was as follows:
MS m/z (ESI): 476 [M+1]
$^1$H NMR (400 MHz, $CD_3OD$) δ 7.51 - 7.36 (m, 6H), 7.29 (d, *J*=8.0 Hz, 1H), 5.21 (s, 2H), 5.13 (dd, *J*=13.4, 5.2 Hz, 1H), 4.51 - 4.38 (m, 3H), 4.13 (d, *J*=8.1 Hz, 1H), 3.78 (dd, *J*=12.2, 5.9 Hz, 2H), 3.46 (d, *J*=8.1 Hz, 1H), 3.09 (dd, *J*=10.4, 1.5 Hz, 1H), 2.95 - 2.84 (m, 1H), 2.76 (ddd, *J*=17.6, 4.6, 2.4 Hz, 1H), 2.60 - 2.43 (m, 2H), 2.20 - 2.11 (m, 1H), 1.88 (d, *J*=10.0 Hz, 1H), 1.68 (d, *J*=10.0 Hz, 1H), 1.38 (d, *J*=6.4 Hz, 3H).
The characterization data of **19** was as follows:
MS m/z (ESI): 476 [M+1]
$^1$H NMR (400 MHz, $CD_3OD$) δ 7.50 - 7.37 (m, 6H), 7.30 - 7.26 (m, 1H), 5.20 (s, 2H), 5.13 (dd, *J*=13.3, 5.2 Hz,

1H), 4.43 (dd, *J*=30.0, 19.2 Hz, 3H), 4.07 (d, *J*=7.9 Hz, 1H), 3.72 - 3.64 (m, 2H), 3.60 (dd, *J*=7.9, 1.8 Hz, 1H), 2.94 - 2.84 (m, 1H), 2.80 - 2.71 (m, 2H), 2.54 - 2.43 (m, 2H), 2.15 (dtd, *J*=12.7, 5.2, 2.3 Hz, 1H), 1.92 (dd, *J*=9.4, 2.3 Hz, 1H), 1.73 (d, *J*=10.0 Hz, 1H), 1.31 (d, *J*=6.5 Hz, 3H).

## Example 14

**5-(4-(*(R)*-1-(4-(((2-(*(S)*-2,6-dioxopiperidin-3-yl)-1-oxoisoindolin-4-yl)oxy)methyl)ph enyl)ethyl)piperazin-1-yl)-2-cyanopyridine (Compound 21)**

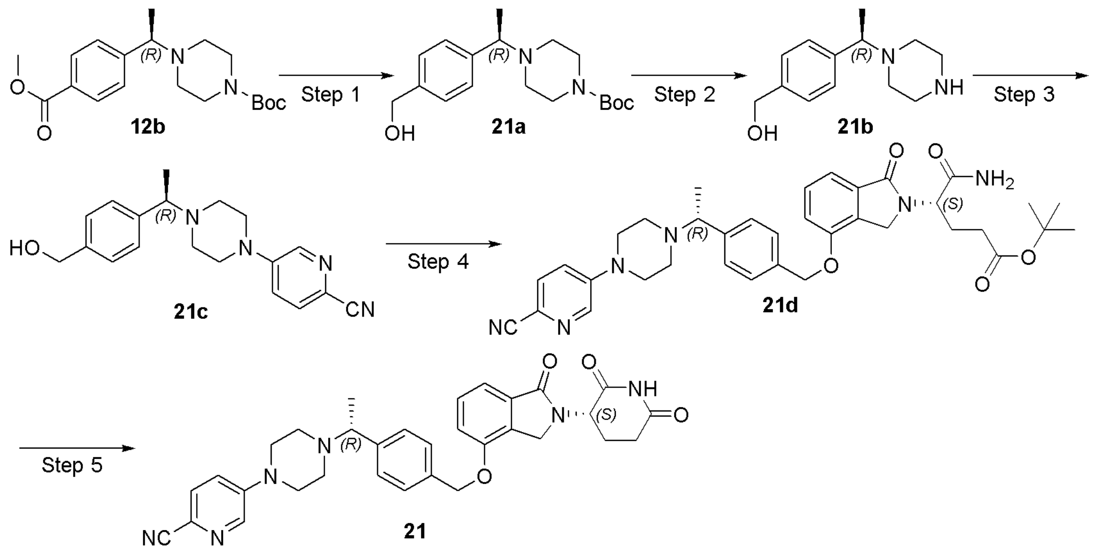


Step 1

Tert-butyl *(R)*-4-(1-(4-(hydroxymethyl)phenyl)ethyl)piperazin-1-carboxylate (**21a**)

To a solution of 12b (2.7 g, 7.75 mmol) in THF (30 mL) was added LAH (353 mg, 9.30 mmol) at 0°C. The mixture was stirred at 0°C for 1 hour, and quenched with water (0.5 mL). Then a NaOH solution (2.5 N, 4 mL) was added. The resulting mixture was diluted with THF (60 mL), and dried over anhydrous sodium sulfate. After filtering, the filtrate was concentrated to dryness under a reduced pressure to produce the target product **21a** (2.75 g, crude product). 1.97 g of the crude product was purified with a silica gel column chromatography (petroleum ether/ethyl acetate=100/0 to 1/4) to produce the target product **21a** (1.75 g).
MS m/z (ESI): 321 [M+1]

Step 2

*(R)*-(4-(1-(piperazin-1-yl)ethyl)phenyl)methanol dihydrochloride (**21b**)

**21a** (1.75 g, 5.46 mmol) was dissolved in 1,4-dioxane (20 mL). Then a solution of hydrogen chloride in 1,4-dioxane (4 M, 20 mL) was added. The mixture was stirred at room temperature for 18 hours, and the solvent was removed under a reduced pressure. The residue was purified with a reversed-phase preparative high performance liquid chromatography to produce the target product **21b** (560 mg, 35%).
MS m/z (ESI): 221 [M+1]
$^1H$ NMR (400 MHz, DMSO-$d_6$) δ 12.37 (s, 1H), 9.76 (s, 1H), 9.52 (s, 1H), 7.60 (d, *J*=7.9 Hz, 2H), 7.41 (d, *J*=8.0 Hz, 2H), 4.60 (s, 1H), 4.53 (s, 2H), 4.18 (s, 2H), 3.84 (s, 1H), 3.69 - 3.29 (m, 4H), 3.05 (d, *J*=51.8 Hz, 2H), 1.71 (d, *J*=6.7 Hz, 3H).

Step 3

*(R)*-5-(4-(1-(4-(hydroxymethyl)phenyl)ethyl)piperazin-1-yl)-2-cyanopyridine (**21c**)

**21b** (110 mg, 0.38 mmol), 2-cyano-5-fluoropyridine (51 mg, 0.42 mmol), cesium carbonate (371 mg, 1.14 mmol) and DMF (4 mL) were mixed. The mixture was heated to 60°C, stirred for 2 hours, and cooled to room temperature. The reaction mixture was filtered through Celite. The filtrate was concentrated to dryness under a reduced pressure. The residue was purified by a silica gel column chromatography (dichloromethane/methanol=100/0 to 19/1) to produce the target product **21c** (24 mg, 52%).
MS m/z (ESI): 323 [M+1]

Step 4

Tert-butyl *(S)*-5-amino-4-(4-((4-(*(R)*-1-(4-(6-cyanopyridin-3-yl)piperazin-1-yl)ethyl)benzyl)oxy)-1 -oxoisoindolin-2-yl)-5 -oxopentanoate (**21d**)

**21c** (64 mg, 0.20 mmol) was dissolved in dichloromethane (10 mL). Then thionyl chloride (48 mg, 0.40 mmol) was added. The mixture was stirred at room temperature for 2 hours, and concentrated to dryness under a reduced pressure. The residue was dissolved in DMF (4mL). Then **5f** (67 mg, 0.20 mmol) and potassium carbonate (138 mg, 1 mmol) were added. The mixture was heated to 50°C, stirred for 20 hours, cooled to room temperature and filtered. The filtrate was concentrated to dryness under a reduced pressure. The residue was purified by a silica gel column chromatography (dichloromethane/methanol=100/0 to 19/1) to produce the target product **21d** (92 mg, 72%).
MS m/z (ESI): 639 [M+1]

Step 5

5-(4-(*(R)*-1-(4-(((2-(*(S)*-2,6-dioxopiperidin-3-yl)-1-oxoisoindolin-4-yl)oxy)methyl)phen yl)ethyl)piperazin-1-yl)-2-cyanopyridine formate (**21**)

**21d** (156 mg, 0.29 mmol), TsOH·$H_2O$ (110 mg, 0.58 mmol) and acetonitrile (10 mL) were mixed. The mixture was heated to 90°C, stirred for 20 hours, cooled to room temperature, and adjusted with a saturated sodium bicarbonate solution to pH=8. Then the resulting mixture was extracted with ethyl acetate (3×50 mL). The combined organic phases were successively washed with saturated sodium bicarbonate solution (50 mL) and a saturated saline solution (50 mL), and then dried over anhydrous sodium sulfate. After filtering, the filtrate was concentrated to dryness under a reduced pressure. The residue was purified with a reversed phase-preparative high performance liquid chromatography to produce the target product **22** (62 mg, solid, containing 0.65 equivalent of formic acid, 45%).
MS m/z (ESI): 565 [M+1]
$^1H$ NMR (400 MHz, DMSO-$d_6$) δ 10.95 (s, 1H), 8.37 (d, *J*=2.9 Hz, 1H), 8.16 (s, 0.65H), 7.72 (d, *J*=8.9 Hz, 1H), 7.48 (dd, *J*=16.8, 8.1 Hz, 3H), 7.38 - 7.28 (m, 5H), 5.23 (s, 2H), 5.11 (dd, *J*=13.3, 5.1 Hz, 1H), 4.42 (d, *J*=17.4 Hz, 1H), 4.26 (d, *J*=17.5 Hz, 1H), 3.48 (q, *J*=6.6 Hz, 1H), 3.37 (t, *J*=4.9 Hz, 4H), 2.96 - 2.85 (m, 1H), 2.56 (dd, *J*=15.0, 10.7 Hz, 3H), 2.48 - 2.38 (m, 3H), 1.98 (dt, *J*=10.2, 5.0 Hz, 1H), 1.33 (d, *J*=6.7 Hz, 3H).
Compound **22** was prepared according to the experimental procedures from step 1 to step 5 for compound **21,** but in step 3, 6-chloro-5-fluoronicotinonitrile was used to replace 2-cyano-5-fluoropyridine.

| Compound No. | Compound structure | Compound in replacement of 2-cyano-5-fluoropyr idine | MS m/z (ESI) |
|---|---|---|---|
| **22** | 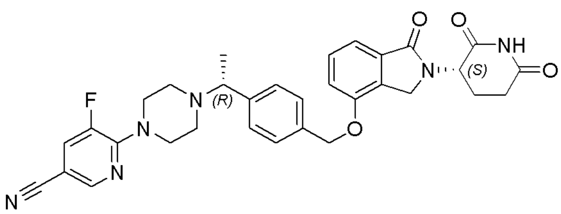 | 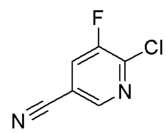 | 583 |

The NMR data of Compound **22** was as follows:

| Compound | $^1$H NMR |
|---|---|
| 6-(4-((*R*)-1-(4-(((2-((*S*)-2 ,6-dioxopiperidin-3-yl)-1 -oxoisoindolin-4-yl)oxy) methyl)phenyl) ethyl)pip erazin-1-yl)-5-fluoronico tinonitrile hemiformate (**22**) | $^1$H NMR (400 MHz, $CD_3OD$) δ 8.26 (t, *J*=1.4 Hz, 1H), 8.19 (s, 0.5H), 7.65 (dd, *J*=13.8, 1.8 Hz, 1H), 7.47 (dd, *J*=7.8, 6.0 Hz, 3H), 7.41 (t, *J*=7.2 Hz, 3H), 7.29 (d, *J*=8.0 Hz, 1H), 5.24 (s, 2H), 5.13 (dd, *J*=13.3, 5.2 Hz, 1H), 4.45 (q, *J*=17.3 Hz, 3H), 3.77 (t, *J*=5.0 Hz, 4H), 3.66 (dd, *J*=13.4, 6.7 Hz, 1H), 2.96 - 2.82 (m, 1H), 2.77 (qd, *J*=6.9, 3.7 Hz, 3H), 2.70 - 2.58 (m, 2H), 2.49 (ddd, *J*=26.4, 13.2, 4.7 Hz, 1H), 2.16 (dtd, *J*=7.7, 5.4, 2.5 Hz, 1H), 1.50 (t, *J*=9.7 Hz, 3H). |

**Biological assays**

**Example 15**

**Determination for inhibition of the proliferation of NCI-H929 cells**

The effect of the compounds of the present invention on the proliferation of NCI-H929 human myeloma cells was evaluated by using a luminescent cell viability test experiment. The experiment method was summarized as follows: The compound was dissolved and diluted to 5 mM in DMSO, and then serially diluted 4-fold in DMSO to a minimum concentration of 0.31 μM. Each concentration was further diluted 50-fold with RPMI 1640 medium (Thermo Fisher, Cat. No. 72400-047). If the $IC_{50}$ value of the compound was relatively low, the initial concentration of the compound could be lowered.

NCI-H929 cells (Nanjing Cobioer, Cat. No. CBP60243) were cultured in RPMI 1640 complete medium [containing 10% FBS (GIBCO, Cat. No. 10099-141) and 100 units/mL penicillin-streptomycin (Thermo Fisher, Cat. No. 15140122)]. Cells (15000 cells/mL) were plated in 90 μL of the complete medium in a 96-well plate and cultured overnight, and then 10 μL of the compound solution was added into each well. The cells were cultured in an incubator at 37 °C and 5% $CO_2$ for 6 days. Then the cell culture plate was taken out and balanced to room temperature according to the instruction of the CellTilter-Glo (CTG) kit (Promega, Cat. No. G7572). 50 μL CTG reagent was added for complete lysis, the plate was placed at room temperature for 10 minutes, and the luminescence signal was read with a microplate reader (EnVision, Perkin Elmer). The group containing 0.2% DMSO medium was used as 0% inhibition. The XLfit software was used to plot the compound inhibition curve and calculate the inhibitory $IC_{50}$ value. The experiment results were shown in Table 1.

**Example 16**

**Determination of the influence on the secretion of TNFα and IL-2 in cells from human peripheral blood mononuclear cells**

The immunomodulatory function of the compound of the present invention was evaluated by detecting its effect on the secretion amount of TNFα and IL-2 in cells from the human peripheral blood mononuclear cells (hPBMC) by the enzyme linked immunosorbent assay (ELISA) method.

The experiment method was summarized as follows:
The peripheral blood of healthy volunteers was collected by using EDTA anticoagulant tubes, and diluted with phosphate buffered saline (PBS) containing 2% fetal bovine serum (Gibco, Cat. No. 10099-141) in an equal volume ratio. 30 mL of the resulting diluted sample was transferred into a Sepmate-50 centrifuge tube (Stemcell, Cat. No. 86450) to which 15 mL of a density-gradient centrifugation liquid (Sigma, Cat. No. 10771) had been added in advance, and centrifuged at room temperature at 1200×g for 10 minutes. The liquid containing PBMC in the upper layer was transferred to a new 50-mL centrifuge tube, and centrifuged at room temperature at 300×g for 8 minutes. The supernatant was discarded, and the resulting PBMC was resuspended to $5\times10^6$/mL by using RPMI 1640 medium (Gibco, Cat. No. 72400-047). 80μL of the resulting suspension was added to each well in a 96-well plate.

The compound was dissolved and diluted to 5 mM in DMSO (if the $IC_{50}$ value of the compound was relatively low, the initial concentration of the compound could be lowered), and then serially diluted 4-fold in DMSO to a minimum concentration of 0.31 μM. Each concentration was further diluted 50-fold with RPMI 1640 medium. 10 μL of each compound solution was added into the cells in the above 96-well plate. The 96-well plate was placed in an incubator, and the cells were cultured at 37 °C and 5% $CO_2$ for 1 hour. 10 μL of LPS (Sigma, Cat. No. L-2880) with a concentration of 100 ng/mL was added, and the cells were continuously cultured in the incubator at 37 °C and 5% $CO_2$ overnight.

Supernatants were collected for the detection of TNFα.

The above-mentioned 96-well plate can also be placed in an incubator, and the cells were cultured at 37 °C and 5% $CO_2$ for 1 hour. 10 μL of anti-CD3 antibody (Thermo Fisher, Cat. No. 14-0037-82) with a concentration of 500 ng/mL was added, and the cells were continuously cultured in the incubator at 37 °C and 5% CO2 for 72 hours. Supernatants were collected for the detection of IL-2.

TNFα and IL-2 were detected respectively according to each instruction of the ELISA kits (R&D, Cat. No. DY210 and DY202, respectively) to obtain the OD450 values for each well. The group containing 0.2% DMSO medium was used as 0% inhibition or stimulation. The XLfit software was used to plot the compound inhibition or stimulation curve and calculate the corresponding $IC_{50}$ or $EC_{50}$. The experiment results were shown in Table 1.

**Table 1**

| Compound No. | $IC_{50}$ (nM) for the inhibition of the NCI-H929 cell proliferation | $IC_{50}$ (nM) for the inhibition of the TNFa secretion | $EC_{50}$ (nM) for stimulation of the IL-2 secretion |
|---|---|---|---|
| 1 | 7.7 | | |
| 2 | 35 | 7.8 | 10 |
| 3 | 7.0 | 1.1 | 8.0 |
| 4 | 5.0 | 0.9 | 4.1 |
| 5 | 7.8 | | |
| 6 | 0.1 | 2.8 | 2.3 |
| 7 | 0.2 | 2.8 | 3.2 |
| 8 | 0.5 | | 4.0 |
| 9 | 1.1 | | |
| 10 | 11 | 2.1 | 46 |
| 11 | 2.2 | | |
| 12 | 0.3 | | |
| 13 | 1.2 | | |
| 14 | 1.4 | | |
| 15 | 0.4 | 1.7 | 19 |
| 16 | 0.1 | 1.3 | 5.5 |
| 17 | 0.3 | 3.7 | |
| 18 | 3.1 | | 11 |
| 19 | 3.8 | 0.8 | 11 |
| 20 | 0.1 | | 1.9 |
| 21 | 0.04 | 0.5 | 3.8 |
| 22 | 0.6 | 4.7 | 26 |

**Example 17**

**Determination for the blockade of the hERG potassium ion channel**

The effect of the compound of the present invention on possible arrhythmias was assessed by determining the blockade of the hERG potassium ion channel.

The experiment method was summarized as follows:
Extracellular fluid: 140 mM NaCl, 3.5 mM KCl, 1 mM $MgCl_2$, 2 mM $CaCl_2$, 10 mM D-glucose, 10 mM HEPES, 1.25 mM $NaH_2PO_4$, pH=7.4.

Electrode internal solution: 20 mM KCl, 115 mM K-aspartate, 1 mM $MgCl_2$, 5 mM EGTA, 10 mM HEPES, 2 mM $Na_2$-ATP, pH=7.2.

Compound solution: the compound to be tested was dissolved in DMSO and prepared into a stock solution having a concentration of 10 mM, then diluted into the concentration of 3 mM with DMSO, and then diluted into a solution having a concentration of 3 μM with the extracellular solution for subsequent use.

Cell culture: the HEK293 cell line with the stable hERG potassium channel overexpression (Creacell, Cat. No. A-0320) was cultured in a DMEM medium (Gibco, Cat. No. 11995-065) containing 10% fetal calf serum (Gibco, Cat. No. 1428478) and 0.8 mg/mL G418 (Amresco, Cat. No. E859-5G) at the culture temperature of 37°C under the carbon dioxide concentration of 5%. The spent medium was removed and the cells were washed once with PBS (Gibco, Cat. No. 1009-141). Then 1 mL TrypLE™ Express solution (Gibco, Cat. No. 12604021) was added and the cells were incubated at 37 °C for 30 seconds. When the cells were detached from the bottom of the dish, 5 mL of complete medium preheated at 37 °C was added. The cell suspension was transferred to a sterile centrifuge tube and centrifuged at 1000 rpm for 5 minutes before cell collection. Cells were inoculated in 6 cm cell culture dishes at the cell inoculation volume of $2.5*10^5$ per dish (final volume: 5 mL). Before the patch clamp detection experiment, $3*10^3$ cells were plated on the cover slips, cultured in 24-well plates (final volume: 500 μ L), and detected 18 hours later.

Voltage stimulation protocol by recording the whole-cell hERG potassium current with the whole cell patch clamp: after forming a whole-cell seal, the cell membrane voltage was clamped at -80 mV. The clamping voltage was depolarized from -80 mV to -50 mV and maintained for 0.5 seconds (as leakage current detection), then stepped to 30 mV and maintained for 2.5 seconds, then quickly returned to -50 mV and maintained for 4 seconds to excite the hERG channel tail current (Peak tail current), the hERG potassium current was recorded every other 10 seconds. Experiment data were collected with EPC-10 amplifier (HEKA) and stored in PatchMaster (HEKA v2x73) software. Determination: A capillary glass tube (Sutter Instruments) was drawn into a recording electrode with a microelectrode drawing instrument (Sutter Instruments). The cell-loaded cover slip was removed from the 24-well plate placed in the incubator and then placed under an inverted microscope. The electrode internal solution was filled into the recording electrode, then a microelectrode controller (Sutter Instruments) was operated to contact the recording electrode with the cell surface. The negative pressure suction was performed to form the GΩ seal; then the fast capacitance compensation was performed; then, the negative pressure suction was continuously applied until the cell membrane was broken through suction, and the whole cell recording mode was completed. In the whole-cell recording mode, the slow capacitance compensation was performed and the membrane capacitance and the series resistance were recorded, during which the leakage compensation was not applied. After the hERG tail current recorded with the whole cell was stabilized for 3-5 minutes, 8 mL of the compound-free extracellular fluid (blank control) and 8 mL of 3 μM solution of the compound to be tested were sequentially flowed through the recording bath with the gravity perfusion to act on the cells for 5 minutes (or to the current stabilization). The current detected for each cell in the compound-free extracellular fluid was served as its own control. 2-3 cells were detected in independent replicates. All electrophysiological experiments were performed at room temperature.

Data analysis: firstly, the current for which the compound to be tested had been applied was normalized to the blank control current ( $\frac{peak\ tail\ current\ compound}{peak\ tail\ current\ vehicle}$ ), then the inhibition rate corresponding to this compound concentration was calculated, ( $1-\frac{peak\ tail\ current\ compound}{peak\ tail\ current\ vehicle}$ ), and the results were shown in Table 2.

**Table 2**

| Compound No. | The percentage of the inhibition to the hERG current at the solubility of 3 μM (n=2) |
|---|---|
| 6 | 52% |
| 16 | 58% |
| 21 | 49% |
| 22 | 20% |
| CC-92480 | 76% |

**Example 18**

**In vivo pharmacokinetic experiment in rats**

The compound to be tested was dissolved in 5% DMA+20% Solutol+75% Saline vehicle to prepare a 0.5 mg/mL administration solution.

2 mL/kg of the administration solution was administered through the intravenous injection (IV) at a dose of 1 mg/kg to three fed male Sprague-Dawley rats, respectively. Blood samples were collected at 0.083, 0.25, 0.5, 1, 2, 4, 8 and 24 hours after administration. 10 mL/kg of the administration solution was administered by gavage (PO) at a dose of 5 mg/kg to other three fed male Sprague-Dawley rats, respectively. Blood samples were collected at 0.25, 0.5, 1, 2, 4, 8 and 24 hours after administration.

The concentration of the compound to be tested in plasma was obtained with the LC-MS/MS quantitative analysis with an API-4500 mass spectrometer, and the limit of quantitation (LOQ) for the plasma was 1 ng/mL. Pharmacokinetic (PK) parameters were calculated by using WinNonlin and the results were summarized in Table 3.

**Table 3**

| Compound | IV (1 mpk) (n=3) | | PO (5 mpk) (n=3) | | |
|---|---|---|---|---|---|
| | CL (mL/min/kg) | $V_{ss}$ (L/kg) | $C_{max}$ (ng/mL) | $AUC_{last}$ (h*ng/mL) | F (%) |
| 3 | 61 | 4.2 | 105 | 270 | 20 |
| 5 | 110 | 3.3 | 1.6 | 8.8 | 4 |
| 6 | 1.0 | 0.27 | 1797 | 11797 | 14 |
| 7 | 13 | 2.4 | 121 | 1198 | 19 |
| 16 | 5.2 | 0.56 | 555 | 4275 | 27 |
| 21 | 14 | 0.65 | 318 | 1008 | 18 |
| 22 | 2.2 | 0.36 | 1437 | 13029 | 30 |

## Claims

1. A compound represented by general formula (I), or a pharmaceutically acceptable salt, a stable isotope derivative, or an isomer thereof:

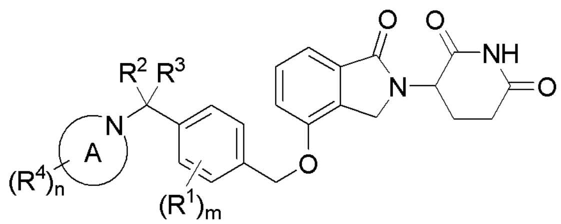

(I)

wherein:

ring A is a nitrogen-containing 4- to 10-membered heterocycle;
$R^1$ is D or halogen;
$R^2$ and $R^3$ are each independently selected from H, D, halogen, cyano, $C_{1-6}$alkyl, $C_{3-6}$cycloalkyl or 3- to 8-membered heterocyclyl, but both cannot be H and/or D at the same time, or $R^2$ and $R^3$ together with the carbon atoms attached thereto form $C_{3-6}$cycloalkane or 3- to 8-membered heterocycle;
$R^4$ is D, halogen, cyano, oxo, $C_{1-6}$alkyl, $C_{3-6}$cycloalkyl, $-OC_{1-6}$alkyl, $-C(O)C_{1-6}$alkyl, $-C(O)C_{3-6}$cycloalkyl, $-S(O)_2C_{1-6}$alkyl, $-S(O)_2C_{3-6}$cycloalkyl, aryl or heteroaryl, wherein one or more hydrogens of the alkyl, the cycloalkyl, the aryl and the heteroaryl are optionally substituted with D, halogen, cyano, $C_{1-2}$alkyl or fluoro$C_{1-2}$alkyl;
m is an integer of 0-4; and
n is an integer of 0-2.

2. The compound according to claim 1 or a pharmaceutically acceptable salt, a stable isotope derivative, or an isomer thereof, wherein ring A is a 4- to 10-membered monocyclic heterocycle containing one N atom, or a fused-, bridged- or spiro-bicyclic heterocycle (such as morpholine, piperidine, thiomorpholine-1,1-dioxide, 2-oxa-5-azabicyclo[2.2.1]heptane, or the like); $R^4$ is D, halogen, oxo, $-CF_3$ or $C_{1-6}$alkyl.

**3.** The compound according to claim 1 or a pharmaceutically acceptable salt, a stable isotope derivative, or an isomer thereof, wherein ring A is a 6- to 10-membered monocyclic heterocycle containing two N atoms, or a fused-, bridged- or spiro-bicyclic heterocycle (such as piperazine, 3,6-diazabicyclo[3.1.1]heptane, 2,6-diazaspiro[3.3]heptane, or the like), preferably ring A is piperazine; $R^4$ is attached to the second N atom and is $C_{1-6}$alkyl, $C_{3-6}$cycloalkyl, $-C(O)C_{1-6}$alkyl, $-C(O)C_{3-6}$cycloalkyl, $-S(O)_2C_{1-6}$alkyl, $-S(O)_2C_{3-6}$cycloalkyl, phenyl, or 5- or 6-membered heteroaryl containing N, O and/or S, wherein one or more hydrogens of the alkyl, the cycloalkyl, the phenyl and the heteroaryl are optionally substituted with D, halogen, cyano, $C_{1-2}$alkyl or fluoro$C_{1-2}$alkyl; n is 0 or 1.

**4.** The compound according to any of claims 1-3 or a pharmaceutically acceptable salt, a stable isotope derivative, or an isomer thereof, wherein $R^2$ is H; $R^3$ is cyano, $C_{1-6}$alkyl or $C_{3-6}$cycloalkyl, preferably $R^3$ is methyl or cyano.

**5.** The compound according to any of claims 1-3 or a pharmaceutically acceptable salt, a stable isotope derivative, or an isomer thereof, wherein both $R^2$ and $R^3$ are methyl.

**6.** The compound according to any of claims 1-3 or a pharmaceutically acceptable salt, a stable isotope derivative, or an isomer thereof, wherein $R^2$ and $R^3$ together with the carbon atoms attached thereto form $C_{3-6}$cycloalkane.

**7.** The compound according to any of the previous claims or a pharmaceutically acceptable salt, a stable isotope derivative, or an isomer thereof, wherein m is 0.

**8.** The compound according to claim 1 or a pharmaceutically acceptable salt, a stable isotope derivative, or an isomer thereof, which is a compound represented by the following general formula (II):

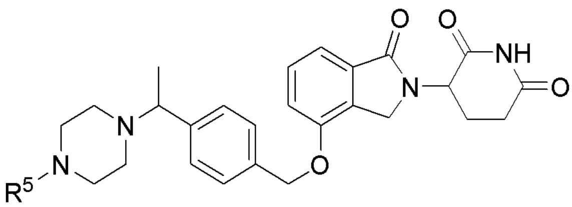
(II)

wherein:
$R^5$ is H, $C_{1-6}$alkyl, $C_{3-6}$cycloalkyl, $-C(O)C_{1-6}$alkyl, $-C(O)C_{3-6}$cycloalkyl, $-S(O)_2C_{1-6}$alkyl, phenyl or 5- to 6-membered heteroaryl containing N, O and/or S, wherein one or more hydrogens of the alkyl, the cycloalkyl, the phenyl and the heteroaryl are optionally substituted with D, halogen, cyano or $C_{1-2}$alkyl, preferably $R^5$ is phenyl, pyridinyl or pyrimidinyl, wherein one or two hydrogens of the phenyl, the pyridinyl and the pyrimidinyl are optionally substituted with F or cyano.

**9.** The compound according to claim 1 or a pharmaceutically acceptable salt, a stable isotope derivative, or an isomer thereof, wherein the compound is selected from:

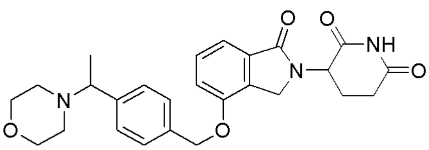 , 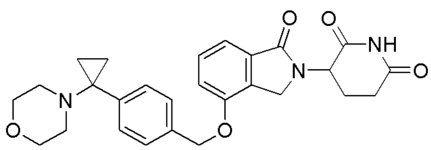 ,

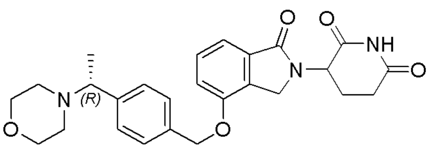 , 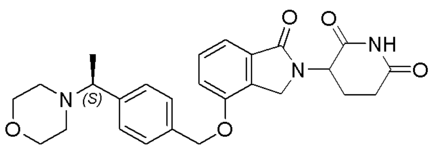 ,

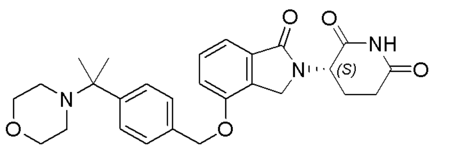 , 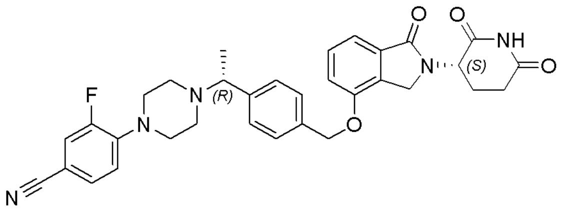 ,

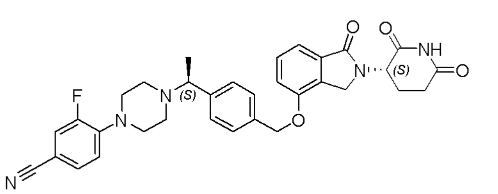 , 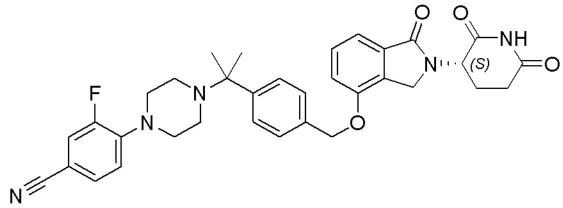 ,

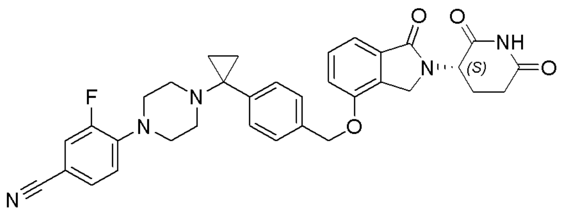 , 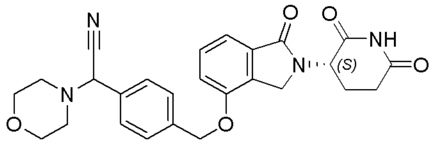 ,

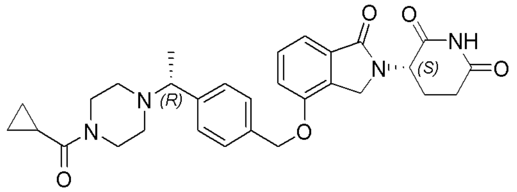 , 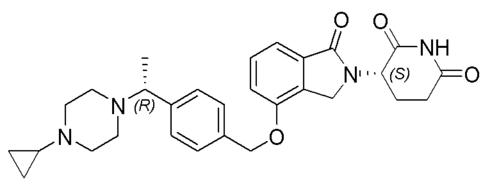 ,

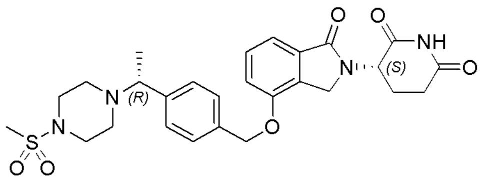 , 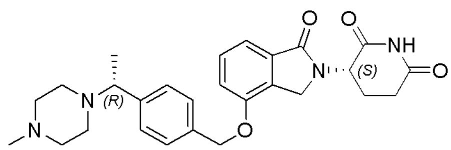 ,

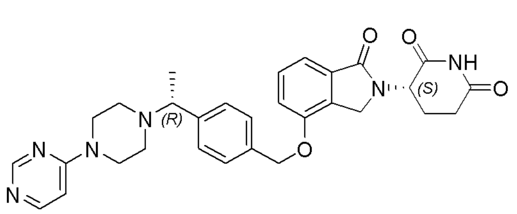 , 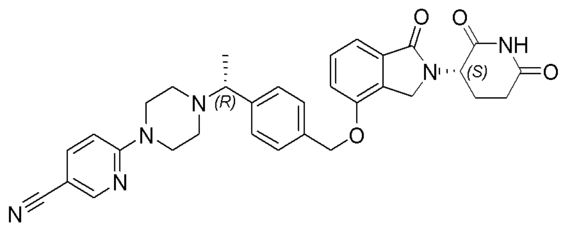 ,

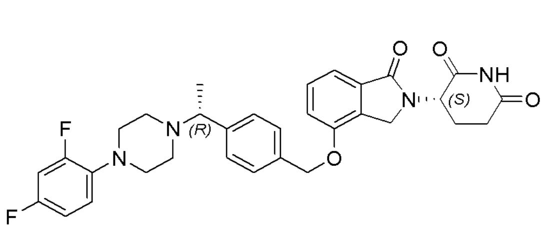 , 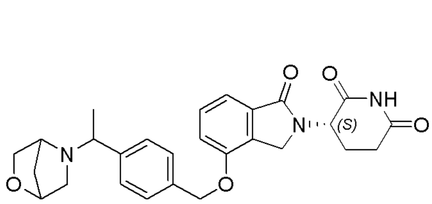 ,

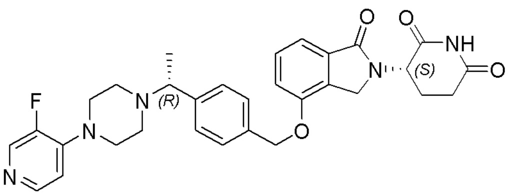 , 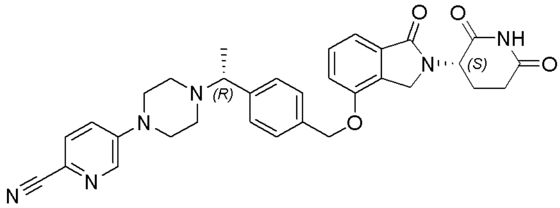 ,

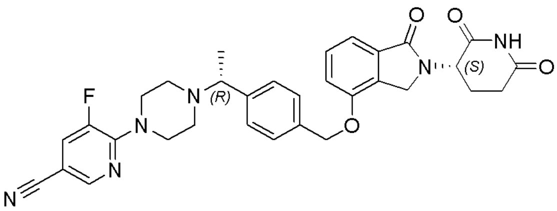 .

**10.** The compound according to claim 9, which is:

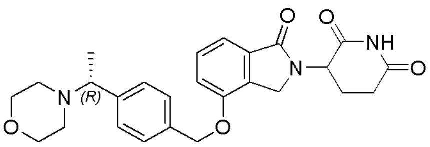 , 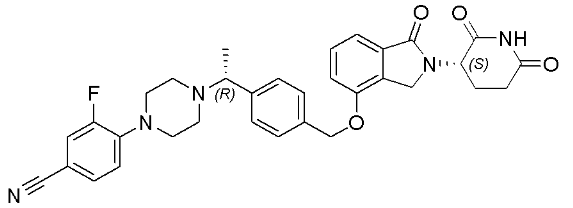 ,

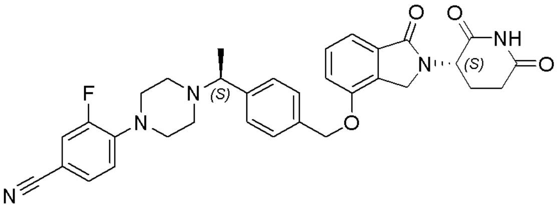 , 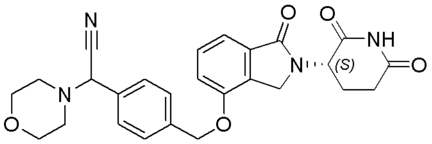 ,

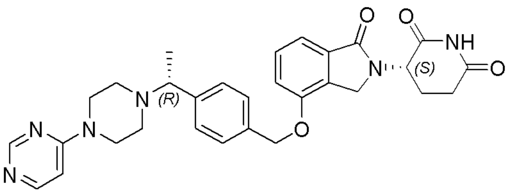 , 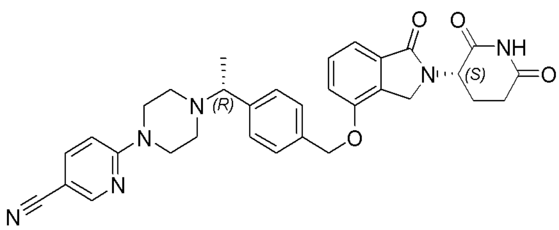 ,

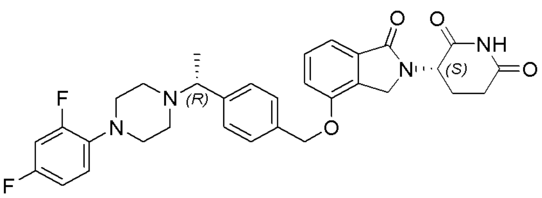 , 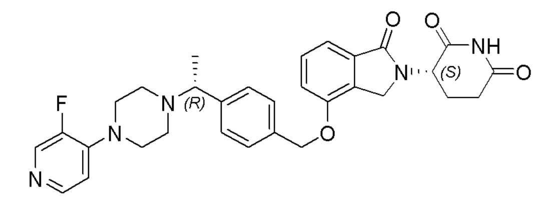 ,

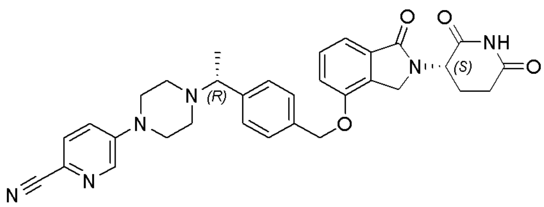 , 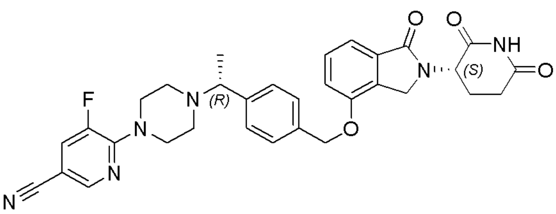 .

**11.** A pharmaceutical composition, which contains the compound according to any of claims 1-10 or a pharmaceutically acceptable salt, a stable isotope derivative, or an isomer thereof and a pharmaceutically acceptable carrier or adjuvant.

**12.** A pharmaceutical composition, which contains the compound according to any of claims 1-10 or a pharmaceutically acceptable salt, a stable isotope derivative, or an isomer thereof and at least one additional drug, wherein said at least one additional drug is selected from chemotherapeutic agents, immune and/or inflammation modulators, neurologically related disease modulators, and the like.

**13.** A method for treating or preventing a correlated disease mediated by Aiolos, Ikaros, Helios, CK1α, GSPT1, IL-2, IL-6, TNFα, IFNy, VEGF or the like, wherein said method comprises administrating a therapeutically effective amount of the compound according to any of claims 1-12 or a pharmaceutically acceptable salt, a stable isotope derivative, or an isomer thereof, or a pharmaceutical composition containing the compound to a subject in need thereof, said disease includes, but is not limited to, hematological tumors, solid tumors, autoimmune diseases, inflammations, neurodegenerative diseases, skin diseases, or the like.

**INTERNATIONAL SEARCH REPORT**

International application No.
**PCT/CN2021/102873**

**A. CLASSIFICATION OF SUBJECT MATTER**

C07D 403/14(2006.01)i； C07D 401/04(2006.01)i； C07D 487/04(2006.01)i； A61P 35/00(2006.01)i； A61K 31/4412(2006.01)i

According to International Patent Classification (IPC) or to both national classification and IPC

**B. FIELDS SEARCHED**

Minimum documentation searched (classification system followed by classification symbols)

C07403/-; C07D401/-; C07D487/-; A61P35/-; A61K31/-

Documentation searched other than minimum documentation to the extent that such documents are included in the fields searched

Electronic data base consulted during the international search (name of data base and, where practicable, search terms used)

WPI, EPODOC, CNKI, Caplus(STN), Registry(STN), heterocyclic, heteroaryl, benzene, tumor, cancer, inflammation, 杂环, 杂芳基, 苯, 肿瘤, 癌症, 炎症

**C. DOCUMENTS CONSIDERED TO BE RELEVANT**

| Category* | Citation of document, with indication, where appropriate, of the relevant passages | Relevant to claim No. |
|---|---|---|
| X | CN 102822165 A (CELGENE CORPORATION) 12 December 2012 (2012-12-12)<br>claims 1, 7, 19-20, description paragraphs [0170]-[0190], paragraph [0233] | 1-13 |
| X | CN 111247138 A (BIOTHERYX INC.) 05 June 2020 (2020-06-05)<br>claims 1, 65-94, abstract | 1-13 |
| A | CN 110506039 A (ARVINAS INC.) 26 November 2019 (2019-11-26)<br>claims 1-16, abstract | 1-13 |
| A | WO 2020023782 A1 (BIOTHERYX, INC.) 30 January 2020 (2020-01-30)<br>entire document | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2021** | **28 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/102873**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

[1] Claim 13 relates to a method for treating a disease. However, the search has been made on the basis of the application of the described compound in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/102873**

| Patent document cited in search report | Publication date (day/month/year) | Patent family member(s) | Publication date (day/month/year) |
|---|---|---|---|
| CN 102822165 A | 12 December 2012 | US 2019135780 A1 | 09 May 2019 |
| | | JP 2013519675 A | 30 May 2013 |
| | | ES 2713482 T3 | 22 May 2019 |
| | | IL 248843 D0 | 31 January 2017 |
| | | US 2020231567 A1 | 23 July 2020 |
| | | SG 183257 A1 | 27 September 2012 |
| | | WO 2011100380 A1 | 18 August 2011 |
| | | US 10669257 B2 | 02 June 2020 |
| | | UA 114856 C2 | 10 August 2017 |
| | | PH 12014501082 B1 | 28 September 2015 |
| | | KR 101931468 B1 | 20 December 2018 |
| | | NZ 601289 A | 31 October 2014 |
| | | NI 201200132 A | 22 April 2013 |
| | | US 2016159768 A1 | 09 June 2016 |
| | | US 2018037567 A1 | 08 February 2018 |
| | | AU 2011215877 C1 | 19 January 2017 |
| | | RS 56232 B1 | 30 November 2017 |
| | | JP 6215976 B2 | 18 October 2017 |
| | | EP 3106460 B1 | 10 April 2019 |
| | | US 2016159772 A1 | 09 June 2016 |
| | | KR 20130010888 A | 29 January 2013 |
| | | KR 20180000338 A | 02 January 2018 |
| | | IL 248843 A | 31 December 2019 |
| | | HU E033009 T2 | 28 November 2017 |
| | | ES 2738776 T3 | 27 January 2020 |
| | | JP 2016172746 A | 29 September 2016 |
| | | CA 2787823 A1 | 18 August 2011 |
| | | RS 58523 B1 | 30 April 2019 |
| | | PT 3202460 T | 05 August 2019 |
| | | RU 2012138709 A | 20 March 2014 |
| | | CR 20120414 A | 31 October 2012 |
| | | SI 3202460 T1 | 30 October 2019 |
| | | EP 2536706 A1 | 26 December 2012 |
| | | HR P20191312 T1 | 18 October 2019 |
| | | CY 1119177 T1 | 14 February 2018 |
| | | JP 2016106106 A | 16 June 2016 |
| | | NZ 717149 A | 30 June 2017 |
| | | EP 3599236 A1 | 29 January 2020 |
| | | AU 2011215877 A1 | 16 August 2012 |
| | | SG 10201501062 S A | 29 April 2015 |
| | | LT 3202460 T | 10 October 2019 |
| | | EP 3202461 A1 | 09 August 2017 |
| | | JP 2017031165 A | 09 February 2017 |
| | | EC SP12012098 A | 30 October 2012 |
| | | KR 101812356 B1 | 26 December 2017 |
| | | DK 3202460 T3 | 29 July 2019 |
| | | US 2013324518 A1 | 05 December 2013 |
| | | SI 3202461 T1 | 31 May 2019 |
| | | US 8518972 B2 | 27 August 2013 |
| CN 111247138 A | 05 June 2020 | US 10927104 B2 | 23 February 2021 |

Form PCT/ISA/210 (patent family annex) (January 2015)

**INTERNATIONAL SEARCH REPORT**
**Information on patent family members**

International application No.
**PCT/CN2021/102873**

| Patent document cited in search report | Publication date (day/month/year) | Patent family member(s) | Publication date (day/month/year) |
|---|---|---|---|
| | | US 2019062320 A1 | 28 February 2019 |
| | | CA 3072543 A1 | 28 February 2019 |
| | | JP 2020531481 A | 05 November 2020 |
| | | EP 3672955 A1 | 01 July 2020 |
| | | WO 2019040274 A1 | 28 February 2019 |
| | | TW 201919639 A | 01 June 2019 |
| | | US 2020231582 A1 | 23 July 2020 |
| | | US 10513515 B2 | 24 December 2019 |
| | | AU 2018321567 A1 | 19 March 2020 |
| CN 110506039 A | 26 November 2019 | IL 272523 D0 | 31 March 2020 |
| | | EP 3660004 A1 | 03 June 2020 |
| | | AU 2017341723 A1 | 04 April 2019 |
| | | CO 2019003642 A2 | 19 June 2019 |
| | | CA 3038979 A1 | 19 April 2018 |
| | | EP 3526202 A4 | 29 April 2020 |
| | | KR 20190055260 A | 22 May 2019 |
| | | KR 102173463 B1 | 04 November 2020 |
| | | KR 20200020978 A | 26 February 2020 |
| | | US 2018099940 A1 | 12 April 2018 |
| | | IL 265806 D0 | 30 June 2019 |
| | | JP 2019530715 A | 24 October 2019 |
| | | MX 2019004278 A | 05 August 2019 |
| | | AU 2020201792 B2 | 03 September 2020 |
| | | JP 2020100649 A | 02 July 2020 |
| | | RU 2019113229 A | 02 November 2020 |
| | | CN 111892577 A | 06 November 2020 |
| | | US 2021040044 A1 | 11 February 2021 |
| | | US 2020095205 A1 | 26 March 2020 |
| | | WO 2018071606 A1 | 19 April 2018 |
| | | US 10844021 B2 | 24 November 2020 |
| | | AU 2020201792 A1 | 26 March 2020 |
| | | RU 2019113229 A3 | 02 November 2020 |
| | | US 10584101 B2 | 10 March 2020 |
| | | EP 3526202 A1 | 21 August 2019 |
| | | US 2021009528 A1 | 14 January 2021 |
| WO 2020023782 A1 | 30 January 2020 | TW 202019936 A | 01 June 2020 |
| | | AU 2019309894 A1 | 28 January 2021 |
| | | US 2020048277 A1 | 13 February 2020 |
| | | CA 3106239 A1 | 30 January 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107739389
- WO 2014025978 A